(19) Europäisches Patentamt
European Patent Office
Office européen des brevets

(11) **EP 1 996 695 B1**

(12) **FASCICULE DE BREVET EUROPEEN**

(45) Date de publication et mention de la délivrance du brevet:
**30.10.2013 Bulletin 2013/44**

(21) Numéro de dépôt: **07731753.5**

(22) Date de dépôt: **16.03.2007**

(51) Int Cl.:
*C12N 1/14* *(2006.01)*     *C12N 9/34* *(2006.01)*
*C12N 9/62* *(2006.01)*     *C12P 7/06* *(2006.01)*

(86) Numéro de dépôt international:
**PCT/FR2007/050937**

(87) Numéro de publication internationale:
**WO 2007/113417 (11.10.2007 Gazette 2007/41)**

(54) **COMPLEMENT NUTRITIONNEL POUR MILIEU DE FERMENTATION ALCOOLIQUE**

NAHRUNGSERGÄNZUNG FÜR EIN ALKOHOLISCHES GÄRUNGSMITTEL

NUTRITIONAL SUPPLEMENT FOR ALCOHOLIC FERMENTATION MEDIUM

(84) Etats contractants désignés:
**AT BE BG CH CY CZ DE DK EE ES FI FR GB GR
HU IE IS IT LI LT LU LV MC MT NL PL PT RO SE
SI SK TR**

(30) Priorité: **17.03.2006 FR 0602398**

(43) Date de publication de la demande:
**03.12.2008 Bulletin 2008/49**

(73) Titulaire: **Etablissements J. Soufflet
10400 Nogent Sur Seine (FR)**

(72) Inventeurs:
• **BARET, Jean-Luc
F-77250 Veneux Les Sablons (FR)**
• **LABEILLE, Pierre
F-14450 Blainville/Orne (FR)**

(74) Mandataire: **Colas, Jean-Pierre
Cabinet Lavoix
2, Place d'Estienne d'Orves
75441 Paris Cedex 09 (FR)**

(56) Documents cités:
**EP-A1- 1 022 329**

• LIN YAN ET AL: "Ethanol fermentation from biomass resources: current state and prospects" APPLIED MICROBIOLOGY AND BIOTECHNOLOGY, vol. 69, no. 6, février 2006 (2006-02), pages 627-642, XP002407201 ISSN: 0175-7598
• HARBHAJAN SINGH: "Fungal Treatment of Distillery and Brewery Wastes" MYCOMEDIATION: FUNGAL BIOREMEDIATION. ONLINE ISBN: 9780470050590, 2006, pages 76-114, XP002450806
• LABEILLE P ET AL: "Comparative study of wheat flour saccharification and ethanol production with two glucoamylase preparations" INDUSTRIAL CROPS AND PRODUCTS, ELSEVIER, NL, vol. 6, no. 3/4, 1 août 1997 (1997-08-01), pages 291-295, XP002119202 ISSN: 0926-6690

**Description**

La présente demande décrit un complément nutritionnel favorisant la production industrielle d'éthanol par fermentation alcoolique suivant notamment un procédé de saccharification-fermentation simultanées, à partir de matière première glucidique fermentescible en éthanol, éventuellement après saccharification, telle qu'une matière première amylacée, en particulier de céréales, notamment le blé, et de coproduits (drêches, son). La demande décrit également des drêches issues de ce procédé et un procédé de fabrication de levure en aérobiose.

Il est connu de produire de l'éthanol à partir d'une matière première amylacée par un procédé enzymatique comprenant une étape de liquéfaction de l'amidon par une alpha-amylase visant à solubiliser et hydrolyser l'amidon en dextrines. Cette étape est classiquement suivie d'une étape de saccharification par une glucoamylase, aussi appelée amyloglucosidase, visant à hydrolyser les dextrines en glucose. La teneur en glucose en fin de saccharification est alors sensiblement de 100% de l'équivalent en glucose correspondant à l'amidon de ladite matière amylacée. Autrement dit, tout le potentiel en glucose de la matière amylacée utilisée a été transformé en glucose.

Le glucose est alors fermenté au cours d'une étape de fermentation pendant laquelle il est transformé en éthanol par une levure dans des conditions anaérobies.

Un axe de recherche pour améliorer ce type de procédé enzymatique a consisté à tenter de fusionner les étapes de fermentation et de saccharification. Les chercheurs ont ainsi essayé de réaliser la fermentation et la saccharification simultanément au cours d'une étape unique appelée « saccharification-fermentation », en anglais « simultaneous saccharification and fermentation » ou SSF. La teneur en glucose du milieu au début de cette étape unique, c'est-à-dire en sortie de liquéfaction, est alors typiquement inférieure à 3 % de l'équivalent en glucose correspondant à l'amidon de ladite matière amylacée.

Des essais ont également été effectués pour limiter la durée de l'étape de saccharification de manière que la teneur en glucose en sortie de cette étape soit inférieure à 100% de l'équivalent en glucose correspondant à l'amidon de ladite matière amylacée. L'étape de saccharification est alors qualifiée de « pré-saccharification » ou de « saccharification partielle ».

Comme on le verra plus en détail dans la suite de la description, les procédés de fabrication d'éthanol comportant une étape de saccharification incomplète, voire pas d'étape de saccharification, ci-après appelés « procédés à saccharification incomplète », impliquent des contraintes spécifiques. Des solutions techniques adoptées dans le cadre de procédés conventionnels avec saccharification complète avant fermentation ne peuvent donc pas a priori être utilisées avec ces procédés.

Dans le cadre des procédés à saccharification incomplète, on appelle « milieu de saccharification-fermentation », un milieu où s'effectuent la fermentation et au moins une partie de la saccharification.

L'article lin et Tanaka (2006) Appl. Microbiol. Biotechnnol. 69:627-642 décrit l'utilisation de la saccharification-fermentation simultanée dans des procédés de fermentation alcoolique. Néanmoins, la saccharification-fermentation simultanée est uniquement suggérée dans des procédés de fermentation à partir de matières premières cellulosiques et non amylacées.

La demande européenne EP 1 022 329 décrit un procédé de fabrication d'éthanol à partir de matières premières amylacées comprenant une étape de fermentation en présence d'un produit multienzymatique, ledit produit multienzymatique étant introduit pendant l'étape d'hydrolyse de l'amidon, appelée "étape de saccharification", qui est une étape préliminaire à la fermentation alcoolique par une levure. Il ne suggère pas l'utilisation de ce produit multienzymatique au cours d'une saccharification-fermentation simultanée.

Il existe un besoin permanent d'améliorer le rendement en éthanol des procédés de production d'éthanol, notamment à saccharification incomplète.

La présente invention a pour objet de répondre à ce besoin.

Selon l'invention, on atteint ce but au moyen d'un complément nutritionnel pour milieu de saccharification-fermentation dans un procédé de fabrication d'éthanol par fermentation à partir d'une matière première amylacée comportant, et de préférence étant constitué, par un principe actif issu d'une fermentation avec une moisissure. Il peut être sous forme liquide ou solide.

La présente invention concerne ainsi l'utilisation d'un complément nutritionnel dans la fabrication d'éthanol à partir d'une matière première amylacée par saccharification-fermentation,
le complément nutritionnel étant un complément nutritionnel pour milieu de fermentation alcoolique à partir de matières premières glucidiques, comportant un principe actif, sous forme brute ou extraite, issu de la fermentation d'un son de blé avec une moisissure choisie parmi les souches *d'Aspergillus niger* ATCC 201202, ATCC 76060, ATCC 76061, MUCL 28815, MUCL 28816, NRRL 3112 ou parmi les souches *d'Aspergillus orizae* ATCC 22788 et ATCC 42149.

Comme on le verra plus en détail dans la suite de la description, la présence d'un tel complément nutritionnel dans un milieu de saccharification-fermentation s'est avérée particulièrement avantageuse pour la croissance et l'efficacité de la levure. Il en est résulté une amélioration significative du rendement du procédé de fabrication de l'éthanol à partir d'une matière première amylacée.

De préférence, le procédé décrit dans la demande comporte une, et de préférence encore plusieurs, des caractéristiques optionnelles suivantes :

- Le complément nutritionnel est un mélange, solide ou liquide, dudit principe actif et d'au moins une enzyme utile. La notion d'enzyme utile inclut notamment une ou des enzymes ayant une activité amylasique, notamment glucoamylasique, protéolytique ou xylanasique, et leurs combinaisons.

- Le principe actif présent dans le complément nutritionnel décrit ici est sous forme brute, c'est-à-dire qu'il comporte le substrat mis en oeuvre lors de la fermentation avec ladite moisissure, ou extraite, c'est-à-dire qu'il est isolé dudit substrat. Il est éventuellement en mélange avec des enzymes additionnelles. De préférence, le substrat est un son de blé.

- Le principe actif est choisi dans le groupe formé par l'ergostérol, la N-acétylglucosamine, les vitamines, en particulier vitamine B, les acides nucléiques, les acides aminés et, de préférence, leurs mélanges. Parmi les acides aminés, on vise notamment un ou plusieurs, de préférence l'ensemble des acides aminés suivants : alanine, arginine, asparagine, acide aspartique, valine, leucine, acide glutamique. Parmi les vitamines, on note la présence de différentes vitamines B, par exemple B1, B2, B3, B6 et inositol, de la vitamine E, entre autres.

- Le complément nutritionnel présente, lors de son introduction dans le milieu de saccharification-fermentation, notamment du fait d'un choix approprié de ladite moisissure, les activités enzymatiques suivantes :

    o glucoamylasique : au moins 500 UG, de préférence 750 UG, de préférence encore 1500 UG par gramme de matière sèche, et/ou de préférence,
    o protéolytique : au moins 100 UP, de préférence au moins 400 UP par gramme de matière sèche, et/ou, de préférence,
    o xylanasique : au moins 100 UX, de préférence au moins 400 UX par gramme de matière sèche.

- Le complément nutritionnel résulte d'une fermentation, de préférence en milieu solide, d'un substrat, de préférence un son de blé, avec une moisissure, notamment choisie parmi les souches *d'Aspergillus niger,* de préférence choisie parmi les souches ATCC 201202, ATCC 76060, ATCC 76061, MUCL 28815, MUCL 28816, NRRL 3112 ou parmi les souches *d'Aspergillus orizae,* de préférence choisie parmi les souches ATCC 22788 et ATCC 42149.

- Le complément nutritionnel est obtenu au moyen d'un procédé selon l'invention comprenant les étapes suivantes :

    i) prendre du son de blé,
    ii) humidifier, acidifier à un pH compris entre 4 et 5, de préférence avec de l'acide nitrique ($HNO_3$) et traiter thermiquement ledit son de façon à le pasteuriser ou le stériliser, le traitement thermique étant de préférence postérieur à l'humidification,
    iii) inoculer le son de blé résultant par une souche *d'Aspergillus niger,* choisie parmi ATCC 201202, ATCC 76060, ATCC 76061, MUCL 28815, MUCL 28816, NRRL 3112, ou par une souche *d'Aspergillus orizae* choisie parmi ATCC 22788 et ATCC 42149,
    iv) faire fermenter le son de blé à l'état solide dans un réacteur agité périodiquement, à une température de 28 °C à 38°C, la teneur en humidité étant maintenue entre 45% et 65%, de préférence entre 50% et 60% en poids, dans des conditions d'aération propres à éviter une accumulation de dioxyde de carbone permanente au sein du son de blé, jusqu'à ce que le produit de fermentation présente les valeurs minimales suivantes d'activités enzymatiques :

        ■ glucoamylasique : au moins 500 UG, de préférence 750 UG, de préférence encore 1500 UG par gramme de matière sèche et, de préférence,
        ■ protéolytique : au moins 100 UP, de préférence 400 UP par gramme de matière sèche,
        ■ xylanasique : au moins 100 UX, de préférence 300 UX par gramme de matière sèche.

- Le complément nutritionnel est un complexe enzymatique obtenu suivant un procédé décrit dans US 2002 037342.

La demande décrit également un procédé de fabrication d'éthanol à partir d'une matière glucidique, notamment amylacée, comportant, après une étape de liquéfaction, une étape de saccharifcation puis une étape de fermentation, ou après une étape de liquéfaction et éventuellement une étape de pré-saccharification, une étape de saccharification-fermentation dans un milieu de saccharification-fermentation dont la teneur en glucose au début de l'étape de saccharification-fermentation est au plus égal à 95% de l'équivalent en glucose correspondant à la matière glucidique, notamment à l'amidon de ladite matière amylacée. Le procédé décrit ici est remarquable en ce qu'on introduit dans le milieu de fermentation ou de saccharification-fermentation un complément nutritionnel tel que décrit ci-dessus. Cette introduction peut être effectuée directement dans le milieu de fermentation ou de saccharification-fermentation, ou lors d'une étape en amont.

La présente invention concerne également un procédé de fabrication d'éthanol à partir d'une matière glucidique qui est une matière amylacée, comprenant une étape de fermentation en présence d'un complément nutritionnel, et comportant, après une étape de liquéfaction et éventuellement une étape de pré-saccharification, une étape de saccharification-fermentation dans un milieu de saccharification-fermentation dont la teneur en glucose au début de l'étape de saccharification fermentation est au plus égal à 95% de l'équivalent en glucose correspondant à l'amidon de ladite matière amylacée, dans lequel ledit complément nutritionnel est un complément nutritionnel, pour milieu de fermentation alcoolique à partir de matières premières glucidiques, comportant un principe actif, sous forme brute ou extraite, issu de la fermentation d'un son de blé avec une moisissure choisie parmi les souches *d'Aspergillus niger* ATCC 201202, ATCC 76060, ATCC 76061, MUCL 28815, MUCL 28816, NRRL 3112 ou parmi les souches *d'Aspergillus orizae* ATCC 22788 et ATCC 42149,

caractérisé en ce qu'on introduit le complément nutritionnel dans le milieu de saccharification-fermentation.

De manière surprenante, la présence, dans le milieu fermentation ou de saccharification-fermentation, d'un complément nutritionnel tel que décrit ci-dessus, et en particulier d'un son de blé fermenté avec une moisissure, s'est avérée particulièrement favorable pour augmenter l'efficacité de la levure. Celle-ci transforme alors une quantité plus importante de glucose en éthanol avec une cinétique plus favorable.

De préférence, le procédé selon l'invention comporte une, et de préférence encore plusieurs, des caractéristiques optionnelles suivantes :

- La teneur en glucose au début de l'étape de saccharification-fermentation est au plus égal à 3 %, de préférence à 2 %, de préférence encore au plus égal à 1%, de l'équivalent en glucose correspondant à l'amidon de ladite matière amylacée. Autrement dit, le procédé ne comporte pas ni étape de saccharification ni étape de pré-saccharification.

- Le complément nutritionnel étant introduit sous forme brute, la quantité dudit complément nutritionnel est supérieure ou égale à 4 kg de matière sèche, de préférence à 14 kg de matière sèche et/ou inférieure ou égale à 60 kg de matière sèche, de préférence à 34 kg de matière sèche, de préférence encore inférieure à 19 kg de matière sèche par tonne d'amidon, de préférence environ 17 kg de matière sèche par tonne d'amidon.

- Le complément nutritionnel est préparé par fermentation d'un substrat identique à la matière glucidique à laquelle est appliquée le procédé de fabrication d'éthanol.

- L'étape de saccharification-fermentation est entièrement réalisée en l'absence d'apport d'air ou d'oxygène (pas de phase aérobie proprement dite).

- L'étape de saccharification-fermentation comporte une phase initiale aérobie.

- Une levure est utilisée comme agent de fermentation éthanolique, notamment une levure du genre *Saccharomyces*, en particulier *Saccharomyces cerevisiae*. Plus préférablement, la levure est fonctionnelle à des concentrations en éthanol supérieures à 95 g/l.

- Pendant le procédé, ledit complément nutritionnel constitue la principale source nutritionnelle pour la levure et/ou la principale source d'ergostérol et/ou la principale source d'azote et/ou d'acides aminés et/ou de phosphore et/ou de soufre et/ou de vitamines. De préférence il constitue l'unique source d'ergostérol et/ou d'azote et/ou d'acides aminés et/ou de phosphore et/ou de soufre et/ou de vitamines.

Le procédé selon l'invention permet encore de fabriquer des drêches d'une qualité nutritionnelle remarquable. L'invention concerne donc encore des drêches issues d'un procédé de fabrication d'éthanol selon l'invention et comportant plus de 5 g, de préférence plus de 20 g et/ou moins de 100 g, de préférence moins de 35 g, de préférence encore environ 30 g d'ergostérol par tonne de matières sèches de drêches.

De préférence, lesdites drêches comportent encore plus de 40 %, de préférence plus de 50 % de protéines brutes, en pourcentages sur la base de la matière sèche. Comme on le verra dans la suite de la description, de telles teneurs en protéines sont rendues possibles par la consommation d'une partie des hémicelluloses et des fibres au cours de la pré-fermentation ou de la saccharification-fermentation.

La demande décrit enfin, de manière générale, l'utilisation d'un complément nutritionnel tel que décrit ci-dessus pour favoriser une fermentation alcoolique destinée à la fabrication d'éthanol ou une pré-fermentation destinée à la fabrication d'une levure.

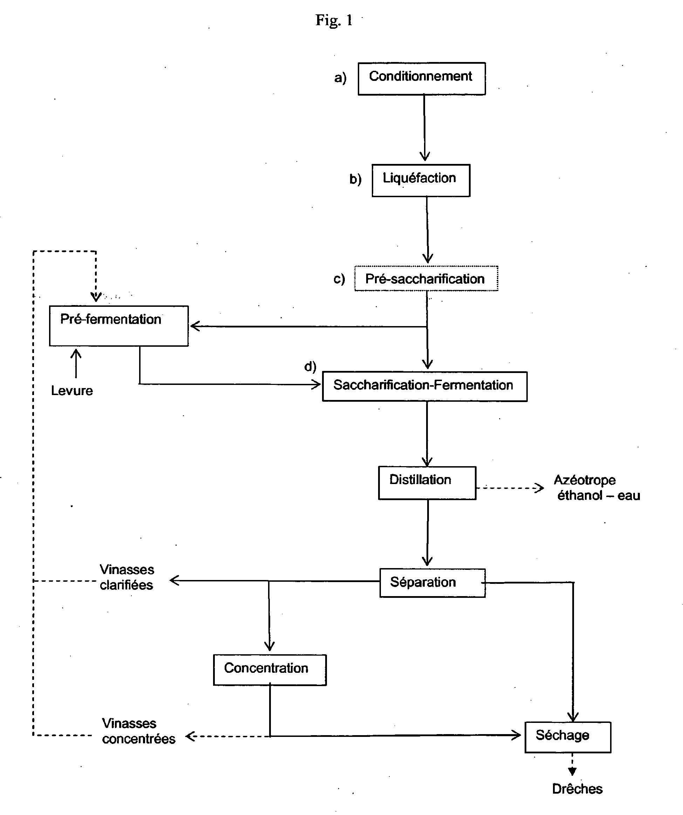

D'autres caractéristiques et avantages de la présente invention apparaîtront encore à la lumière de la description qui va suivre et à l'examen du dessin annexé où la figure 1 représente un schéma d'une partie d'un procédé de fabrication d'éthanol selon l'invention.

Des procédés de f abrication d'éthanol par fermentation de matière glucidiques, notamment amylacée sont bien connus de l'homme du métier, et pourraient être adaptés pour devenir conformes à l'invention. L'homme du métier est donc à même de préciser, le cas échéant, certains points de la description suivante.

Le procédé décrit ci-dessous concerne la fabrication d'éthanol à partir de blé, mais peut s'appliquer à tout type de matière amylacée, en particulier à tout extrait de céréales. Les modes de réalisation décrits ci-dessous ne sont donc que des exemples et on pourrait les modifier, notamment par substitution d'équivalents techniques, sans sortir du cadre de l'invention.

Un procédé de fabrication d'éthanol de blé selon l'invention peut, à titre d'exemple, comporter les étapes suivantes :

a) une étape de conditionnement d'un blé de manière à préparer une mouture d'amidon de blé,

b) une étape de liquéfaction de l'amidon, en particulier en présence d'une alpha-amylase, de manière à hydrolyser l'amidon en dextrines,

c) optionnellement une étape de pré-saccharification, en particulier par un ensemble d'enzymes, de manière à hydrolyser les dextrines en sucres fermentescibles (glucose, maltose, maltotriose) et les constituants non amylacés et

d) une étape de saccharification-fermentation, dans un milieu de saccharification-fermentation contenant les dextrines et/ou lesdits sucres fermentescibles et une levure, de manière à produire de l'éthanol.

Ces différentes étapes sont représentées sur la figure 1.

L'étape a) consiste à préparer une mouture d'amidon de blé, par exemple une farine, par conditionnement dudit blé.

La farine de blé est alors mélangée dans un mélangeur avec de l'eau, optionnellement de la vinasse, de l'acide et une enzyme de liquéfaction, de manière à former un « moût empâté ».

Le moût empâté contient typiquement de 25 à 35 % en masse de matières sèches, de préférence de 30 à 35%. Le pourcentage de matières sèches est déterminé pour limiter les dépenses énergétiques tout en conservant une fluidité satisfaisante. Par souci économique, le pourcentage de matière sèche est le plus élevé possible afin de limiter les coûts de l'évaporation des vinasses dans la suite du procédé. La quantité de farine apportée au mélangeur est régulée soit par un doseur, soit par une bande peseuse.

Selon l'enzyme de liquéfaction utilisée, le pH doit être ajusté avec une solution d'acide, e.g. acide sulfurique. Selon les enzymes utilisées, classiquement des alpha-amylases bactériennes, notamment thermostables, un sel de calcium peut être éventuellement employé. Le débit d'acide est régulé au moyen d'une sonde de pH installée sur le mélange eau/vinasses avant empâteur. Le pH peut classiquement varier entre 5 et 6,5, selon les enzymes utilisées.

La liquéfaction (étape b)) est alors réalisée à une température comprise entre 80°C et 95°C. Le moût empâté peut être porté à cette température par le biais d'une injection directe de vapeur dans le bac de liquéfaction par des tuyauteries ou par un jet-cooker. Dans le deuxième cas, le moût empâté est porté pendant quelques secondes à une température comprise entre 100°C et 150°C au moyen d'une injection de vapeur dans une tuyère avant d'être refroidi rapidement entre 80°C et 95°C. Les bacs de liquéfaction peuvent être agités.

Les caractéristiques préférées de l'étape de liquéfaction sont les suivantes:

o Température : 80°C à 90°C
o pH: 5,5-6,5
o Matière sèche : 30 % à 35 %
o Temps de séjour : 30 minutes à 2 heures.

L'étape b) conduit à l'hydrolyse de l'amidon en dextrines.

En cas de pré-saccharification (étape c)), le moût liquéfié est refroidi dans des échangeurs thermiques de type échangeurs à plaques ou échangeurs tubulaires à une température comprise entre 50°C et 60°C.

Dans certains cas, une dilution du moût liquéfié peut être effectuée avec un diluant tel que l'eau ou des vinasses de recyclage ou les flegmasses provenant de la distillerie.

A l'étape c), étape optionnelle, le moût liquéfié est de préférence mis en présence d'un complexe enzymatique présentant les activités enzymatiques souhaitées.

Les débits des enzymes sont de préférence asservis au débit de moût entrant, à la concentration en glucose produit et à la teneur en amidon restant. L'objectif de cet asservissement est de préparer une solution dépourvue d'amidon en fin de fermentation.

Les caractéristiques préférées de l'étape c) de pré-saccharification sont les suivantes :

o. Température : 50° à 60°C
o pH:4à5
o Temps de séjour: jusqu'à obtention de la teneur en glucose recherchée, généralement inférieur à 24h.

Les bacs de pré-saccharification sont soumis à une agitation mécanique permettant une bonne homogénéisation du moût en cours de saccharification et par là même une mise en contact facilitée entre les enzymes et les dextrines à hydrolyser.

L'utilisation du complexe enzymatique issu de la fermentation de son de blé par une moisissure permet avantageusement de réduire la viscosité du moût saccharifié et d'augmenter la concentration en azote dans ledit moût.

Selon l'invention, la saccharification n'est pas poursuivie jusqu'à hydrolyse totale des dextrines en glucose, mais interrompue alors que l'hydrolyse n'est que partielle. Selon l'invention le taux de glucose à la fin de l'étape de pré-saccharification est inférieur à 95%, de préférence inférieur à 50% de préférence encore inférieur à 5%. L'hydrolyse se poursuit donc durant l'étape de saccharification-fermentation.

Si le procédé selon l'invention ne comporte pas d'étape de saccharification partielle, ou " pré-saccharification ", la teneur en glucose au début de l'étape d) de saccharification-fermentation est au plus égal à 3 %, de préférence à 2 %, de préférence encore au plus égal à 1%, de l'équivalent en glucose correspondant à l'amidon de ladite matière amylacée.

A l'étape d), le moût est mis en présence d'une levure dans le milieu de saccharification-fermentation.

In est aussi mis en présence d'un complément nutritionnel tel que décrit ci-dessus. L'ordre d'incorporation de la levure et du complément n'a pas d'importance.

L'ensemble est agité pendant toute la conduite de l'étape d).

Toutes les levures utilisées pour la production d'éthanol peuvent être utilisées, en particulier des levures du genre *Saccharomyces*. Habituellement, la levure est introduite dans le milieu de saccharification-fermentation dans des conditions aérobies. Pendant cette phase, la levure accomplit un certain nombre de divisions cellulaires. La levure ne transforme pas alors le glucose en éthanol, mais au contraire consomme du glucose pour sa croissance. Pour améliorer cette croissance cellulaire, il est connu d'apporter au milieu de saccharification-fermentation des compléments nutritionnels. Par ailleurs, afin de limiter la croissance cellulaire, dont la consommation de glucose afférente diminue le rendement de production d'éthanol, il est préférable d'ensemencer le milieu de saccharification-fermentation avec une quantité de levure telle que la production d'éthanol par les levures permet rapidement d'atteindre des concentrations d'éthanol dans le milieu de culture s'opposant à la croissance cellulaire de la levure.

Suivant une première modalité, l'étape d) débute en aérobiose le temps nécessaire à une multiplication suffisante de la levure.

Le milieu de saccharification-fermentation est ensuite placé sous conditions anaérobies. La levure transforme alors le glucose en éthanol.

Suivant une deuxième modalité, l'étape d) est entièrement conduite en anaérobiose. La présence du complément nutritionnel selon l'invention permet de se passer de la phase initiale aérobie.

Les caractéristiques préférées de l'étape d) de saccharification-fermentation sont les suivantes :

o Température : de 30°C à 35°C
o pH : ajusté au début de l'étape d) avec de l'acide (e.g. acide sulfurique) entre environ 3,5 et environ 5, de préférence entre environ 3,8 et environ 5, de préférence encore entre environ 4 et environ 5, mieux encore entre environ 4 et environ 4,5.
o Après ajustement du pH au début de l'étape d), grâce à l'effet tampon du complément nutritionnel, on ne prévoit pas de régulation du pH pendant la suite de l'étape d)
o inoculum de levure : environ $10^6$ à environ $5.10^8$ UFC de levure (unités formant colonies) par ml de milieu de saccharification-fermentation, de préférence environ $10^7$ UFC de levure par ml de milieu de saccharification-fermentation.
○ 20 % à 35%, notamment 20 % à 30% de MS
○ Temps de séjour : 20 heures à 72 heures, notamment 20 heures à 60 heures. Le temps de séjour augmente avec la MS.

Le milieu de saccharification-fermentation selon l'invention contient un complément nutritionnel tel que décrit ci-dessus. Ce complément peut être introduit directement dans le milieu de saccharification-fermentation ou lors d'une étape en amont. De manière surprenante, l'ajout dans le milieu de saccharification-fermentation d'un tel complément nutritionnel, notamment d'un son de blé fermenté avec une moisissure, s'est avéré particulièrement favorable pour augmenter l'efficacité de la levure. Celle-ci transforme alors une quantité plus importante de glucose en éthanol avec une cinétique plus favorable. En outre, le complément nutritionnel tel que décrit ci-dessus permet de réduire la durée de la phase aérobie en début d'étape de saccharification-fermentation, voire de la supprimer. Avantageusement, le rendement en est amélioré. Avantageusement encore, le complément nutritionnel tel que décrit ci-dessus permet de diminuer la mortalité cellulaire par rapport à une culture identique conduite en l'absence dudit complément nutritionnel. Enfin, sa présence a un effet tampon évitant de devoir réguler le pH.

Selon l'invention, de préférence, le milieu de saccharification-fermentation contient initialement, pour 1 000 kg d'amidon introduits initialement, entre 2,5 kg et 35 kg de complément nutritionnel, notamment son de blé fermenté, en particulier entre 8 et 10 kg de complément nutritionnel, notamment son de blé fermenté, pour 1 000 kg d'amidon.

La présence dans le milieu de saccharification-fermentation du son de blé fermenté permet donc de réduire considérablement le temps de saccharification-fermentation.

Il semblerait que ce complément nutritionnel apporte des nutriments parfaitement adaptés à la levure, en des proportions et sous une forme optimales, en particulier pour les cultures anaérobies.

Sans être liés par une théorie, les inventeurs expliquent ces résultats de la manière suivante. Ils ont découvert la présence, dans le complément nutritionnel utilisé selon l'invention, d'acides aminés nécessaires à la levure. Ces acides aminés contribueraient ainsi à la nutrition azotée de la levure de manière très efficace, notamment beaucoup plus efficace que les simples sels d'ammonium utilisés jusqu'ici, réduisant en outre la synthèse de coproduits fermentés tels que le glycérol et améliorant de fait le rendement en éthanol.

La présence de vitamines dans le complément nutritionnel tel que décrit ci-dessus permettrait aussi d'expliquer une meilleure efficacité de la levure.

Enfin les inventeurs ont découvert que le complément nutritionnel tel que décrit ci-dessus contient de l'ergostérol et de la N-acétylglucosamine, qui sont des constituants importants de la levure. Leur présence dans le milieu de saccharification-fermentation aide à la croissance et au bon fonctionnement de la levure en anaérobiose complète. Dans ces conditions, sa synthèse est en effet impossible par la levure. La présence d'ergostérol permet donc avantageusement de réduire, voir de supprimer la phase aérobie en début de saccharification-fermentation.

Les essais du tableau 1 ci-dessous ont cependant démontré que l'ajout des nutriments identifiés ne conduit pas à un milieu de saccharification-fermentation aussi performant que l'ajout d'un complément nutritionnel selon l'invention. C'est donc bien la combinaison de l'ensemble des constituants de ce complément nutritionnel, dans les proportions résultant de la fermentation avec une moisissure, et de préférence leur présentation sous forme d'un son de blé, qui sont à l'origine des performances exceptionnelles obtenues.

Le complément nutritionnel peut ne pas présenter d'activités enzymatiques particulières ou des activités enzymatiques optimales. Des ajouts enzymatiques sont alors nécessaires.

De préférence cependant, le complément nutritionnel tel que décrit ci-dessus présente également des activités enzymatiques utiles dans le cadre du procédé de fabrication de l'éthanol. En particulier, il est préférable que le complément nutritionnel présente une activité glucoamylasique supérieure à 500 UG par gramme de matière sèche. De préférence encore le complément nutritionnel présente une activité protéolytique supérieure à 100 UP par gramme de matière sèche et/ou une activité xylanasique d'au moins 100 UX par gramme de matière sèche.

Ainsi, bien que l'utilisation d'autres enzymes tels que des enzymes purifiées ou des complexes d'enzymes purifiées soit envisageable, il est préférable, selon l'invention, d'utiliser un complément nutritionnel fermenté dans des conditions lui permettant de présenter lesdites activités enzymatiques. De préférence, ce complément nutritionnel est alors introduit à l'étape d), et/ou le cas échéant, à l'étape c), comme complexe enzymatique.

De préférence, le complément nutritionnel est un son de blé fermenté préparé ou susceptible d'être obtenu suivant le procédé selon l'invention suivant :

i) prendre du son de blé,

ii) humidifier et traiter thermiquement ledit son de façon à le pasteuriser ou le stériliser, le traitement thermique étant de préférence postérieur à l'humidification,

iii) inoculer le son de blé résultant par une souche *d'Aspergillus niger,* choisie parmi ATCC 201202, ATCC 76060, ATCC 76061, MUCL 28815, MUCL 28816, NRRL 3112, de préférence parmi ATCC 76061 et NRRL 3112, de préférence encore la souche ATCC 76061 ou parmi les souches d'Aspergillus orizae ATCC 22788 et ATCC 42149.

iv) faire fermenter le son de blé à l'état solide, de préférence sous la forme d'une couche de plus de 10 cm d'épaisseur, dans un réacteur agité périodiquement, à une température de 28°C à 38°C, la teneur en humidité étant maintenue entre 45% et 65%, de préférence entre 50% et 60% en poids, dans des conditions d'aération propres à éviter une accumulation permanente de dioxyde de carbone au sein du son de blé, jusqu'à ce que le produit de fermentation présente les valeurs d'activités enzymatiques minimales suivantes:

■ glucoamylasique : au moins 500 UG, de préférence 750 UG, de préférence encore 1500 UG par gramme de matière sèche et, de préférence,

■ protéolytique : au moins 100 UP, de préférence 400 UP par gramme de matière sèche, et de préférence encore,

■ xylanasique : au moins 100 UX, de préférence 400 UX par gramme de matière sèche.

A l'étape i), le son de blé est préférentiellement choisi de manière à avoir une proportion d'au moins 40 % en masse de particules inférieures à 1 mm.

A l'étape ii), le son de blé doit être humidifié et traité thermiquement en vue de le pasteuriser ou le stériliser. Il est avantageux que le traitement thermique ne précède pas l'humidification, car on a observé des résultats de fermentation médiocres dans le cas où on traite thermiquement le son avant de l'humidifier.

Le traitement thermique peut consister en un chauffage par exemple dans un autoclave. Un traitement en autoclave de 20 minutes entre 120 et 121 °C s'est avéré très satisfaisant. Des conditions moins sévères de pasteurisation à 105°C, pendant 15 minutes dans une étuve conviennent aussi. Il est possible également d'opérer le traitement thermique du son en y injectant de la vapeur d'eau, ce qui permet d'opérer simultanément à l'humidification du son.

De préférence, on règle le pH, de préférence avec de l'acide nitrique, lors de l'humidification dans une gamme de 4 à 5,5 afin d'améliorer l'effet pasteurisant du traitement thermique et le démarrage de la fermentation souhaitée. L'utilisation d'acide nitrique est particulièrement avantageuse, l'acide nitrique étant également utilisé comme source d'azote pour la moisissure.

Outre sa fonction de stérilisation, le traitement thermique a pour effet de favoriser la gélatinisation de l'amidon contenu dans le son de blé et, donc la disponibilité de ce substrat pour les champignons *Aspergillus niger* et *Aspergillus orizae*, ce qui permet des fermentations plus efficaces.

L'humidification du son est importante car la teneur en eau influe sur la performance de la fermentation. Elle est déterminée de manière que la teneur en eau du son soit, au début de l'étape iv) de fermentation, dans la plage 50-60%, de préférence 50-55%, de la masse totale du son et de l'eau.

A l'étape iii), l'inoculation du son de blé peut se pratiquer avec tout inoculum approprié. L'homme du métier connaît de multiples façons de préparer un inoculum convenable à partir d'une souche sélectionnée. La dose d'inoculation est avantageusement d'au moins $10^7$ spores/gramme de matière sèche initiale.

A l'étape iv), la fermentation peut être conduite dans tout réacteur approprié. Des exemples de réacteurs utilisables sont ceux décrits dans l'article de A. DURAND et Coll. publié dans Agro-Food-Industry Hi-Tech (Mai-Juin 1997, pages 39-42).

La fermentation doit être conduite jusqu'à ce que l'activité glucoamylasique soit d'au moins 500 UG, de préférence d'au moins 750 UG, de préférence encore d'au moins 1500 UG par gramme de matière sèche de son, c'est-à-dire normalement pendant une période de 1 à 3 jours, de préférence de 30 à 60 heures. En dessous de 1 jour, la fermentation est trop incomplète. Au bout de 3 jours la fermentation est achevée ou quasiment achevée de sorte qu'il serait non économique de la prolonger davantage.

La température du milieu est maintenue entre 28°C et 38°C, de préférence entre 32°C et 36°C, ce qui correspond au domaine d'activité optimum connu des souches utilisées. Avantageusement, à cette fin, la température de l'air est réglée entre 34°C et 38°C pendant les premières heures de la fermentation pour favoriser la germination des spores, puis réduite entre 28°C et 32°C pour le reste de la fermentation pour contribuer à la régulation de la température du milieu.

La teneur en humidité du son de blé est normalement comprise entre 50% et 60%. Le taux d'humidité peut cependant s'écarter de +/- 5 unités % de l'intervalle de 50-60% pendant une relativement brève période entre deux ajustements successifs du taux d'humidité ou en fin de fermentation. Il convient, en tout cas, de ne pas descendre en dessous d'un taux d'humidité de 45%. Le taux d'humidité du milieu de culture tend à baisser au cours de la culture par évaporation sous l'effet de l'augmentation de température générée par la croissance fongique, ledit milieu étant un mauvais conducteur de la chaleur. On doit donc maintenir la teneur en humidité pendant la fermentation, par exemple en procédant périodiquement à des apports d'eau pour compenser la perte en eau du milieu. La qualité d'eau utilisée joue aussi un rôle non négligeable. On peut utiliser une eau courante de bonne qualité ou de l'eau distillée.

Le pH du milieu de fermentation n'est pas habituellement régulé. Comme expliqué précédemment il est de préférence ajusté initialement entre 4 et 5.

Si sa valeur de départ est voisine de 6,0-6,4, le pH baisse à 3,8-4,2, au cours de la culture, puis remonte à la fin. Ce retour est généralement corrélé avec la phase de sporulation du champignon. Le suivi de l'évolution du pH constitue un bon indicateur de l'état de la culture.

Le fermenteur doit être aéré, de préférence en continu, afin d'apporter l'oxygène nécessaire à la fermentation et éviter l'accumulation excessive de dioxyde de carbone produit par la fermentation. En outre, l'aération participe au contrôle de la température et de l'humidité du milieu de culture. L'air est, de préférence, sensiblement saturé en eau pour limiter la tendance à l'assèchement du milieu. Il est difficile de donner des indications quantitatives sur le débit d'aération, car de multiples variables, en particulier la taille et la géométrie du réacteur, la quantité du son chargée, etc, interviennent. De simples essais de routine permettront, toutefois, à l'homme de l'art de déterminer aisément un débit d'aération convenable dans chaque cas pratique, généralement un débit d'air de 1 à 2 $m^3$/h par kg de matière sec est

approprié, la suppression est de préférence entre 0,5 et 1 bar.

La charge de son dans le fermenteur doit être périodiquement agitée, au cours de la fermentation à l'aide de moyens d'agitation tels que des bras agitateurs, des lames ou spatules, ou des vis sans fin en vue d'éviter la formation de masses imperméables et afin que l'aération intéresse de la façon la plus homogène possible toute la masse de son. De manière surprenante, les souches mises en oeuvre résistent à l'agitation. Il faut, toutefois, éviter une agitation trop vigoureuse.

Le son de blé fermenté utilisé dans le procédé selon l'invention peut être séché ou congelé en vue de sa conservation, si désiré ou refroidi et utilisé sans transformation supplémentaire.

Le séchage est de préférence réalisé à une température modérée pour ne pas affecter l'activité enzymatique. Un chauffage en étuve à 40°C s'est révélé approprié. Au niveau industriel, de préférence on ventile de l'air sec entre 35 et 45°C, selon la moisissure utilisée. La congélation, de son côté, peut être réalisée sur le produit humide à basse température, par exemple à -20°C.

US 2002 037342 divulgue un complexe enzymatique à activités glucoamylasique, protéolytique et xylanasique obtenu par fermentation de son de blé avec des souches d'*Aspergillus*. Ce complexe peut être utilisé comme complément nutritionnel selon l'invention.

D'une manière générale, on ne peut prévoir l'effet, dans le cadre d'un procédé comportant une étape de saccharification au moins en partie simultanée avec la fermentation, d'une enzyme, ou d'une composition multienzymatique dont l'utilisation est connue dans le cadre d'un procédé à étapes de saccharification et de fermentation séparées. Cela est en particulier vérifié lorsque l'addition d'une composition implique, comme la composition de US 2002 037342, l'incorporation de substances telles que des hémicelluloses susceptibles d'augmenter la viscosité pendant la fermentation.

Ce document décrit l'utilisation de cette composition afin d'améliorer les conditions de l'étape de saccharification. Il ne suggère nullement qu'elle pourrait présenter un intérêt avec un procédé ne comportant pas d'étape de saccharification, ou qu'une étape de pré-saccharification.

En effet, les conditions optimales pour la saccharification et pour la fermentation sont très différentes. En particulier, la saccharification s'effectue couramment à environ 60°C, c'est-à-dire à une température qui ne convient pas aux levures habituellement utilisées pendant la fermentation. Réciproquement, comme illustré par le tableau 7 de US 2002 037342, les enzymes de la composition de US 2002 037342 présentent une activité enzymatique optimale aux températures de saccharification, mais, selon toute attente, présenteraient une activité enzymatique dégradée en cas de saccharification-fermentation simultanées.

En outre, les enzymes de la composition de US 2002 037342 sont connus pour diminuer la viscosité du milieu de saccharification à la température de saccharification (voir tableau 8), mais l'homme du métier pourrait s'attendre à ce que cette diminution de viscosité ne se reproduise pas à la température de fermentation, en tous cas dans une mesure rendant acceptable la viscosité du milieu de saccharification-fermentation.

L'homme du métier n'aurait donc pas tenté d'utiliser la composition de US 2002 037342 dans un procédé de fabrication d'éthanol ne comportant pas d'étape de saccharification, ou qu'une étape de pré-saccharification. D'autant moins que la suppression de la saccharification, ou une limitation de sa durée, peut parfois conduire à un milieu de saccharification-fermentation contaminé de manière réhibitoire, sauf à devoir ajouter de grandes quantités d'agents bactériostatiques. En effet, la température de saccharification permet une protection contre les contaminations bactériennes.

La demande décrit donc également l'utilisation d'un son de blé obtenu selon le procédé décrit précédemment ou conforme au son de blé décrit dans US 2002 037342 pour favoriser la fermentation alcoolique dans un milieu de saccharification-fermentation. De préférence, plus de 4 kg de ce son sont présents dans le milieu de saccharification-fermentation, en début d'étape d) pour 1 000 kg d'amidon.

Avantageusement, dans le mode de réalisation préféré de l'invention, le complément nutritionnel est un complexe multienzymatique obtenu par fermentation d'un son de blé avec une moisissure et :

    o constitue un moyen de valorisation des sons issus de l'étape a) de préparation de la farine de blé,
    o apporte simultanément plusieurs enzymes utiles, simplifiant ainsi le procédé, et
    o est un excellent complément nutritionnel pour la levure.

A ce dernier effet, la quantité massique de complexe multienzymatique doit cependant être beaucoup plus élevée que selon la technique antérieure.

La recherche actuelle tend à fabriquer des complexes multienzymatiques de plus en plus concentrés, afin de limiter les quantités massiques introduites. Les procédés en sont simplifiés.

A contre-courant de cette tendance, les inventeurs ont donc découvert qu'au contraire l'incorporation de plus de 4 kg de matière sèche, et de préférence plus de 14 kg de matière sèche, de préférence encore d'environ 19 kg de matière sèche de son de blé fermenté par tonne d'amidon de départ permet d'améliorer l'efficacité globale du procédé de fabrication de l'éthanol.

A l'issue de l'étape d), le moût fermenté ou « vin » provenant de la saccharification-fermentation, après passage dans un échangeur thermique, alimente des colonnes à distiller.

Les vinasses sortant en pied de colonne sont envoyées à l'atelier de séparation des drêches pour être clarifiées par des méthodes classiques, type centrifugation, afin de séparer des matières solubles des matières insolubles.

Les drêches humides obtenues suite à cette étape de séparation sont composées d'environ 35 % de matière sèche, les vinasses clarifiées sont composées d'environ 7% à 10% de matière sèche.

Les vinasses clarifiées sont concentrées par évaporation sous vide pour obtenir un sirop ou « vinasse concentrées » avec un taux de matière sèche proche de 35%.

Le sirop obtenu peut alors être mélangé aux drêches humides. Le mélange est ensuite séché et on obtient environ 350 kg de drêches ainsi séchées par tonne de blé, le taux de matière sèche de ces drêches étant d'environ 90 %.

Les drêches obtenues après séchage présentent des caractéristiques nutritionnelles remarquables, en particulier pour le bétail, et sont également un objet de l'invention. Les 350 kg de drêches obtenues selon l'invention comportent en effet entre 2 g et 35 g d'ergostérol, de préférence environ 10 g. Or l'ergostérol est un précurseur de la synthèse de la vitamine D2 et présente donc un intérêt pour la santé ainsi qu'un intérêt nutritionnel pour les levures, notamment en anaérobiose.

De plus, dans le cas où le complément nutritionnel est un son de blé fermenté selon le procédé selon l'invention décrit ci-dessus, les drêches obtenues contiennent un surplus de protéines.

Les vapeurs d'alcool des colonnes sont condensées dans des échangeurs thermiques. L'azéotrope récupéré en tête de colonne est séché par des méthodes classiques, par exemple en utilisant des tamis moléculaires.

Le procédé selon l'invention permet d'obtenir environ 375 litres à 390 litres d'éthanol pour une quantité initiale 1 000 kg de blé. Soit, de manière remarquable, jusqu'à 91% du rendement stoechiométrique de la transformation par fermentation du glucose en éthanol pour un blé contenant environ 60% d'amidon.

Sans être liés par cette théorie, les inventeurs expliquent ces résultats par la substitution d'un apport d'azote exogène, par exemple sous forme de sulfate d'ammonium, par de l'azote aminé libre. Cette substitution réduit avantageusement la synthèse de coproduits fermentaires, en particulier de glycérol et donc augmente le rendement.

La demande décrit aussi un procédé de fabrication de levure en aérobiose, ou « propagation de levure », pouvant en particulier être utilisé en pré-fermentation afin de préparer une levure mise en oeuvre dans un milieu de fermentation ou dans un milieu de saccharification-fermentation d'un procédé de fermentation alcoolique du glucose en éthanol.

Dans ce cadre, l'étape de pré-fermentation a généralement pour but d'augmenter la concentration en levure dans le milieu de pré-fermentation d'environ $10^6$ - $10^7$ UFC par ml à au minimum environ $10^8$ UFC par ml de milieu de pré-fermentation, de préférence au minimum environ $5.10^8$ UFC par ml de milieu de pré-fermentation.

La pré-fermentation est effectuée dans des pré-fermenteurs où la température est rigoureusement contrôlée et régulée, par exemple par un système de plaques de refroidissement dans lequel circule un liquide de refroidissement à l'intérieur ou à l'extérieur des cuves de pré-fermentation. Toute multiplication de micro-organismes entraîne en effet une élévation de température qui peut devenir inhibitrice pour la propagation des levures. La température dans les pré-fermenteurs est classiquement maintenue entre 30°C et 35°C.

Un apport en oxygène, par exemple sous forme d'air comprimé, est indispensable à la propagation de la levure.

Afin de favoriser le développement des levures, il est connu de rajouter dans le milieu nutritif :

    o de l'azote apporté sous formes diverses telles que l'urée, l'ammoniac, les sels d'ammonium,
    o du phosphore apporté sous formes diverses telles que l'acide phosphorique, les phosphates,
    o du soufre apporté sous formes diverses telles que l'acide sulfurique, les sulfates,
    o des minéraux essentiels.

Selon la technique antérieure, on ajoute également au milieu nutritif du moût sortant de l'étape de liquéfaction, de saccharification ou de pré-saccharification. Ce moût apporte ainsi des sucres fermentescibles mais conduit à une baisse du rendement global de la fermentation alcoolique. Il existe donc un besoin pour un procédé de fabrication de levure en aérobiose pouvant être utilisé en pré-fermentation afin de préparer une levure mise en oeuvre dans un milieu de fermentation ou dans un milieu de saccharification-fermentation d'un procédé de fermentation alcoolique du glucose en éthanol et qui limiterait cette baisse de rendement.

Comme décrit ici, on atteint ce but en ajoutant dans les pré-fermenteurs au moins une partie des vinasses clarifiées, c'est-à-dire obtenues à l'issue de l'étape de séparation, et/ou des vinasses concentrées obtenues lors de la mise en oeuvre d'un procédé de fabrication d'éthanol par fermentation alcoolique à partir de matières premières glucidiques, notamment amylacées et/ou selon l'invention.

Les vinasses peuvent résulter d'un procédé de fabrication d'éthanol comportant une étape de saccharification-fermentation ou dans lequel les étapes de saccharification et de fermentation sont complètement séparées.

De préférence, les vinasses résultent d'un procédé de fabrication d'éthanol selon l'invention, dans lequel un complément nutritionnel tel que décrit ci-dessus a été introduit.

La présente demande concerne également un procédé de fabrication de levure en aérobiose dans un pré-fermenteur, caractérisé en ce qu'on ajoute dans ledit pré-fermenteur des vinasses obtenues lors de la mise en oeuvre d'un procédé de fabrication d'éthanol selon l'invention.

Ainsi, l'invention concerne également un procédé de fabrication d'éthanol tel que défini ci-dessus, dans lequel on utilise, pour la fermentation éthanolique, des levures obtenues par un procédé de fabrication de levure tel que défini ci-dessus, lequel procédé utilise des vinasses provenant dudit procédé de fabrication d'éthanol.

L'utilisation des vinasses résultant d'un procédé de fabrication d'éthanol selon l'invention, dans lequel un complément nutritionnel selon l'invention a été introduit, pour la production de levure au sein des pré-fermenteurs, est particulièrement avantageuse puisque cela permet de disjoindre la production d'éthanol par les levures d'une part et la croissance cellulaire des levures d'autre part. En effet, les vinasses résultant d'un procédé de fabrication d'éthanol selon l'invention constituent un milieu nutritionnel permettant de faire croître les levures jusqu'à un niveau compatible avec un ensemencement élevé du milieu de fermentation ou de saccharification-fermentation, ce qui entraîne une utilisation par les levures des sucres fermentescibles présents dans le milieu de fermentation ou de saccharification-fermentation principalement pour la production d'éthanol, et non pour la croissance cellulaire, comme cela a été signalé précédemment. Ainsi, les sucres fermentescibles provenant du substrat glucidique de départ sont principalement utilisés pour la production d'éthanol, tandis que ce sont principalement des sucres non fermentescibles qui servent à la croissance cellulaire. Le rendement global de production éthanolique à partir du substrat glucidique de départ s'en trouve donc amélioré par rapport aux procédés dans lesquels les sucres fermentescibles sont utilisés par les levures à la fois pour la production d'éthanol et pour la croissance cellulaire.

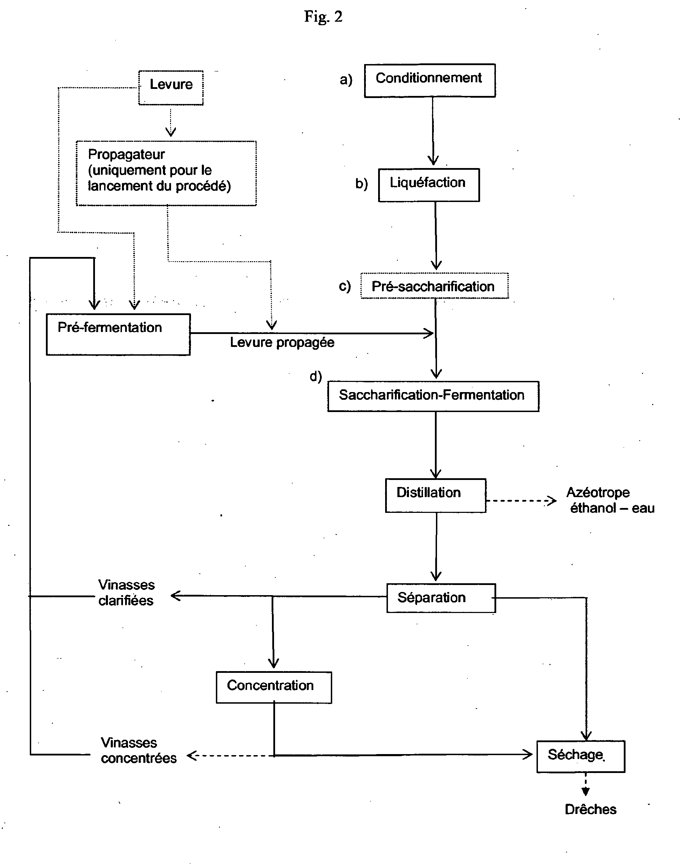

Un mode de réalisation particulier d'un tel procédé est décrit dans la figure 2.

De préférence, le mélange nutritif de pré-fermentation contient des vinasses et est exempt de produits intermédiaires obtenus lors de la mise en oeuvre d'un procédé de fabrication d'éthanol par fermentation alcoolique à partir de matières premières amylacées, en particulier les vinasses ne sont pas mélangées avec du moût issu des étapes intermédiaires d'un procédé de fabrication d'éthanol par fermentation alcoolique à partir de matières premières amylacées.

Suivant une autre modalité préférée, le milieu nutritif de pré-fermentation se compose ou comprend un mélange de vinasses et de moût liquéfié, éventuellement de l'eau.

Suivant encore une autre modalité préférée, le milieu nutritif de pré-fermentation se compose ou comprend un mélange de vinasses et de complément nutritionnel tel que décrit ci-dessus, en particulier son de blé fermenté, éventuellement de l'eau.

Enfin on peut également former un milieu nutritif composé de ou comprenant l'ensemble des éléments précités.

Le milieu nutritif de pré-fermentation selon l'invention contient de préférence une quantité de matière sèche telle que l'agitation et/ou l'aération du milieu est suffisante pour soutenir une production optimale d'éthanol, par exemple de 15% à 20% de matière sèche.

Pour le procédé de fabrication de levure selon l'invention, le complément nutritionnel tel que décrit ci-dessus peut être un son de blé fermenté. Les vinasses issues du milieu de fermentation ou de saccharification-fermentation présentent une composition, et notamment une teneur en sucres utilisables par la levure en aérobiose, particulièrement favorable au développement de la levure dans les pré-fermenteurs. Le milieu de pré-fermentation peut être complété par des éléments nutritifs exogènes comme du glycérol ou des solutions de glycérol ainsi que par des hydrolysats obtenus à partir de drêches humides. Cela permet avantageusement d'économiser du glucose du moût selon l'invention et donc de limiter ladite baisse de rendement.

De préférence au moins une partie des vinasses est recyclée par incorporation dans le milieu nutritif de pré-fermentation. Il est également préférable de recycler les vinasses après concentration afin de limiter la dilution du milieu nutritif du pré-fermenteur.

De préférence, les vinasses présentent dans le milieu nutritif de pré-fermentation se substituent complètement au moût sortant de l'étape de liquéfaction, de saccharification ou de pré-saccharification. Cette substitution peut cependant n'être que partielle.

L'ajout d'un complément nutritionnel tel que décrit ci-dessus, en particulier de son de blé fermenté permet également d'améliorer la pré-fermentation, notamment par enrichissement du milieu nutritif en :

- azote, apporté sous forme d'acides aminés,
- phosphore,
- de soufre,
- vitamines et minéraux essentiels,
- protéines.

Cet ajout peut être effectué directement dans le milieu nutritif de pré-fermentation, ou après mélange avec du moût sortant de l'étape de liquéfaction, ce mélange étant ajouté, après une éventuelle dilution, dans le pré-fermenteur.

L'utilisation de levures pré-fermentées à partir des vinasses issues d'un procédé de fabrication d'éthanol selon l'invention permet également avantageusement d'enrichir considérablement en protéines les drêches séchées obtenues à la fin du procédé de fabrication d'éthanol selon l'invention. Lesdites drêches peuvent alors comporter plus de 40 % de protéines et de préférence au moins 50% de protéines, sur la base de la masse de matière sèche.

Les diverses activités enzymatiques citées dans la description et les revendications ont été mesurées par les méthodes suivantes :

Activité glucoamylasique.

L'action d'une préparation de glucoamylase (GA) sur une solution d'amidon soluble entraîne la libération de sucres réducteurs. Chauffés à 100°C en présence d'acide 3,5-dinitrosalicylique (DNS), ces compositions prennent une couleur brune mesurée au spectrophotomètre (Kontron Instruments, Milan, Italie) à 540 nm.

Le milieu réactionnel contient :

o Solution d'amidon à 1,5% dans le cas d'*Aspergillus niger* et à 2% dans le cas d'*Aspergillus orizae* 1000 $\mu$l
o Tampon citrate 0,1 M à pH 4,5 900 $\mu$l
o Solution enzymatique: 100 $\mu$l

La réaction se déroule pendant 20 minutes à 60°C dans le cas des *Aspergillus niger*, pendant 5 minutes à 50°C dans le cas des *Aspergillus orizae*. Des prélèvements de 100 $\mu$l de milieu réactionnel sont réalisés toutes les 4 minutes dans le cas des *Aspergillus niger* et toutes les minutes dans le cas des *Aspergillus orizae*, mélangés avec 500 $\mu$l de DNS et 400 $\mu$l de tampon citrate à pH 4,5. L'ensemble est ensuite chauffé 5 minutes à 100°C, refroidi rapidement puis dosé à 540 nm face à un blanc d'un mélange de 500 $\mu$l de DNS et de 500 $\mu$l de tampon citrate à pH 4,5.

Ces conditions de dosage ont été établies après avoir étudié l'influence de la température et du pH sur l'activité des préparations de GA. De l'amidon soluble Merck (Darmstadt, Allemagne) a été employé comme substrat de cette hydrolyse enzymatique. Le DNS est préparé selon le protocole proposé par P. Bemfeld, Methods in enzymology, 1, 149-158 (1955) qui est le suivant :

♦ Dissoudre préalablement :

○ 10 g d'acide 3,5-dinitrosalicylique,
○ 200 ml de soude 2 molaires
○ 200 ml d'eau distillée.

♦ Ajouter ensuite :

○ 300 g de tartrate double de sodium potassium.

♦ Compléter le volume à 1 litre avec de l'eau distillée après dissolution totale.

Une fois préparé, ce réactif doit être conservé à l'abri de la lumière. Les courbes d'étalonnage ont été établies avec du glucose comme produit de référence pour le dosage de l'activité glucoamylase ou pour le suivi des réactions de liquéfaction- saccharification, et avec du xylose pour mesurer l'activité xylanase.

Une unité d'activité glucoamylase (UG) correspond à la quantité d'enzyme nécessaire à la libération d'une micromole d'extrémités réductrices par minute dans les conditions du dosage avec le glucose comme référence. L'activité glucoamylase, calculée à l'aide de la formule indiquée ci-dessous, est rapportée à la quantité de matière sèche intiale (MSI):

$$A = \frac{P \times V_{ferm}}{V_{enz} \times M_{ferm}}$$

♦ A correspond à l'activité GA exprimée en UG.gMSI$^{-1}$ ($\mu$mol.min$^{-1}$.gMSI$^{-1}$),
♦ P correspond à la vitesse de libération d'équivalents glucose en $\mu$mol.min$^{-1}$,

♦ $V_{enz}$ représente le volume de la solution d'enzymes dosée en ml,

♦ $V_{ferm}$ est le volume total d'eau distillée utilisé pour extraire la solution d'enzymes en ml,

♦ $M_{ferm}$, exprimée en g de MSI, correspond à la masse initiale de produit sec dont a été extraite la solution d'enzymes.

Activité protéase.

Ce dosage a été mis au point sur de l'azocaséine selon la méthode de Béinon, décrite dans l'ouvrage "Proteins Purification Methods - a Practical Approach", Harris E.L.V. et Angal, S (Editeurs), IRL-Press, Oxford University Press, 1-66 (1989). La dégradation de ce substrat par des protéases entraîne la libération de groupements azo qui absorbent dans l'UV à 340 nm. L'évolution de l'absorbance pendant la cinétique d'hydrolyse de cette protéine indique l'importance de la réaction.

Le milieu réactionnel contient :

o Solution d'azocaséine à 1 %, pH 5,0 : 1 000 $\mu$l

o Solution enzymatique : 200 $\mu$l

L'azocaséine (Sigma, Saint-Louis, Etats-Unis) est dissoute dans un tampon acétate 0,1 mol.l$^{-1}$ à pH 5,0. On a dosé les activités protéases à ce pH car l'azocaséine est insoluble dans le tampon acétate à des pH inférieurs. La réaction enzymatique est réalisée à 60°C. Des prélèvements sont réalisés toutes les 5 minutes pendant 20 minutes et mélangés avec de l'acide trichloroacétique (TCA) à 5 % pour stopper la réaction.

Une unité d'activité protéase (UP) correspond à la quantité d'enzymes nécessaire à l'augmentation de 0,01 unité $A_{340nm}$ par minute, générée par la libération de groupes azo dans les conditions citées précédemment. Cette activité, calculée d'après la formule indiquée ci-dessous, est rapportée à la matière sèche initiale (UP.gMSI$^{-1}$) ou à l'activité glucoamylase (UP.UG$^{-1}$) :

$$A = \frac{P \times V_{ferm}}{V_{enz} \times M_{ferm}}$$

♦ A correspond à l'activité protéase exprimée en UP.gMSI$^{-1}$,

♦ P correspond à la vitesse de libération des groupements azo exprimée en augmentation de 0,01 unité $A_{340nm}$.min$^{-1}$,

♦ $V_{enz}$ représente le volume de la solution d'enzymes dosée en ml,

♦ $V_{ferm}$ est le volume total d'eau distillée utilisé pour extraire la solution d'enzymes en ml,

♦ $M_{ferm}$, exprimée en g de MSI, correspond à la masse initiale de produit sec dont a été extraite la solution d'enzymes.

Activité xylanase

Pour mettre en évidence cette activité enzymatique, on a fait agir les préparations de GA sur une solution de xylane soluble et on a mesuré les sucres réducteurs libérés par la méthode au DNS.

Le milieu réactionnel est composé de :

o Solution de xylane à 2 %, pH 4,5 : 1900 $\mu$l

o Solution enzymatique: 100 $\mu$l

La solution de xylane de mélèze (Sigma à 1 %) est préparée dans du tampon citrate 0,1 M à pH 4,5 et la réaction se déroule à 60°C dans le cas de l'*Aspergillus niger* et à 50 °C dans le cas de l'*Aspergillus orizae*. Des prélèvements de 200 $\mu$l de milieu réactionnel sont effectués toutes les 2 minutes pendant 10 minutes, et mélangés avec 500 $\mu$l de DNS et 300 $\mu$l de tampon citrate à pH 4,5. L'ensemble est ensuite chauffé 5 minutes à 100°C, refroidi rapidement puis dosé à 540 nm face à un blanc d'un mélange de 500 $\mu$l de DNS et de 500 $\mu$l de tampon citrate à pH 4,5.

Une unité d'activité xylanase (UX) correspond à la quantité d'enzymes nécessaire à la libération d'une micromole de sucres réducteurs par minute. Cette activité est rapportée à la matière sèche initiale (UX.gMSI$^{-1}$) ou à l'activité glucoamylase (UX.UG$^{-1}$). Pour calculer cette activité, nous avons repris la formule définie pour le calcul des activités GA dans laquelle :

♦ A correspond à l'activité xylanase exprimée en UX.gMSI$^{-1}$ ($\mu$mol.min$^{-1}$.gMSI$^{-1}$),

♦ P correspond à la vitesse de libération d'équivalents xylose en $\mu$mol.min$^{-1}$,

♦ Les autres termes de la formule ne sont pas modifiés.

Les essais non limitatifs suivants sont donnés dans le but d'illustrer l'invention, en particulier l'avantage que procure l'utilisation d'un son de blé fermenté selon l'invention dans le cas d'un procédé de saccharification-fermentation simultanées.

Exemple 1 : Effet d'une complémentation azotée

On a étudié de façon comparative la quantité d'éthanol produite au cours d'une étape de saccharification-fermentation en introduisant soit une préparation enzymatique commerciale (Spirizyme® Fuel), soit la même préparation enzymatique avec une complémentation non limitante en azote, soit un complément nutritionnel selon l'invention.

Dans ces essais, les inventeurs ont préparé 750 g de moût de farine de blé à 32 % de matière sèche dans un réacteur de 1 litre agité. Après 2 h de liquéfaction à 85°C et pH 5,5, le moût est réparti dans 3 fioles d'Enenmeyer de 0,5 litre contenant chacune 200 g de moût et complété à isoactivité glucoamylase comme suit :

> Fiole 1 : Spirizyme® Fuel (solution de glucoamylase purifiée, Novozymes) ;

> Fiole 2 : Spirizyme® Fuel et complémentation azotée, c'est-à-dire phosphate et sulfate di-ammonique : 3 g/l de chaque produit ;

> Fiole 3 : son de blé fermenté par une souche d'*Aspergillus niger* ATCC 76061, suivant le procédé décrit dans US 2002 037 342.

Le pH initial des moûts dans les trois fioles a été ajusté à 4,5 et la température à 32 °C..

L'incorporation des produits azotés dans la fiole 2 génère une alcalinisation du pH 6,2 nécessitant un apport supplémentaire d'acide chlorhydrique concentré pour ramener le pH initial du moût à 4,5 pour la saccharification-fermentation. Les moûts sont ensemencés avec $10^7$ UFC de levure par ml de moût. La levure utilisée dans les exemples est *Saccharomyces cerevisiae.*

La concentration maximale en glucose théorique dans le moût est de 303 g/l après hydrolyse enzymatique totale de l'amidon.

Comme le montre le tableau 1, la meilleure production d'éthanol est obtenue avec le son de blé fermenté en 72 heures. L'utilisation de Spirizyme® Fuel, même avec un complément azoté ne génère au maximum que 59% d'éthanol en 96 heures. En l'absence d'incorporation d'azote, la production d'éthanol est très faible et nous observons une concentration très élevée en glucose.

Il y a donc un avantage significatif à utiliser du son de blé fermenté par une moisissure pour la production d'éthanol de blé, même à la température du réacteur de saccharification-fermentation simultanées. La comparaison avec le Spirizyme Fuel indique qu'une complémentation non limitante en azote n'explique que partiellement l'amélioration des performances.

Tableau 1

| Durée SSF (h) | Ethanol produit/éthanol final avec le son fermenté (%) | | | Glucose produit/glucose final avec le son fermenté (%) | | |
|---|---|---|---|---|---|---|
| | Spirizyme | Spirizyme +N | son fermenté | Spirizyme | Spirizyme + N | son fermenté |
| 0 | 0,0 | 0,0 | 0,0 | 0,0 | 16,1 | 16,1 |
| 1 | 0,0 | 0,0 | 0,0 | 18,0 | 29,2 | 51,3 |
| 4 | 0,8 | 0,6 | 1,1 | 71,8 | 56,8 | 119,5 |
| 8 | 2,7 | 1,2 | 6,9 | 143,6 | 130,1 | 144,9 |
| 11 | 4,6 | 4,2 | 17,5 | 197,5 | 170,8 | 119,9 |
| 15 | 6,2 | 16,1 | 30,9 | 269,3 | 110,2 | 89,8 |
| 20 | 8,6 | 32,0 | 51,0 | 359,1 | 66,5 | 55,1 |
| 24 | 9,9 | 37,0 | 57,1 | 430,9 | 3,4 | 38,6 |
| 30 | 13,4 | 45,0 | 73,6 | 538,6 | 2,8 | 50,0 |
| 36 | 13,4 | 48,2 | 81,3 | 646,4 | 7,6 | 74,2 |

(suite)

| Durée SSF (h) | Ethanol produit/éthanol final avec le son fermenté (%) | | | Glucose produit/glucose final avec le son fermenté (%) | | |
|---|---|---|---|---|---|---|
| | Spirizyme | Spirizyme +N | son fermenté | Spirizyme | Spirizyme + N | son fermenté |
| 48 | 16,4 | 53,6 | 94,7 | 861,8 | 10,6 | 71,6 |
| 72 | 18,4 | 57,3 | 99,3 | 1292,7 | 133,9 | 81,8 |
| 96 | 16,9 | 59,0 | 100,0 | 1723,6 | 239,0 | 100,0 |

Ces essais confirment l'adéquation du son de blé fermenté pour la production d'éthanol de blé par un procédé de saccharification-fermentation simultanées.

Exemple 2 : Effet tampon du complément nutritionnel selon l'invention.

Cet exemple illustre les avantages de l'utilisation du complément nutritionnel selon l'invention pour favoriser une saccharification-fermentation alcoolique. Cet exemple porte en particulier sur l'effet tampon, du complément nutritionnel selon l'invention, sur le milieu de saccharification-fermentation.

Dans ces essais, les inventeurs ont préparé 750 g de moût de farine de blé à 32 % de matière sèche dans un réacteur de 1 litre sous agitation. Après 1h30 de liquéfaction à 85°C, à pH 5,5 et sous agitation à 250 rpm, le moût est réparti dans 2 fioles d'Erlenmeyer de 250 ml. Chaque fiole contient 100 g de moût liquéfié à 32 % de matière sèche et 40 g d'eau stérile pour obtenir un moût à 23 % de matière sèche. Ensuite on rajoute dans chaque moût :

> Fiole 1 : 0,5 g de son de blé fermenté par une souche d'*Aspergillus* nigerATCC 76061, suivant le procédé décrit dans US 2002 037 342;

> Fiole 2 : 16,7 µl de Spirizyme® Fuel (solution de glucoamylase purifiée, Novozymes) .

Le pH initial des moûts dans les deux fioles est ajusté à 4. Les moûts sont ensemencés avec $5 \times 10^6$ UFC de levures Ethanol Red® (Lesaffre) par ml de moût.

Dans la fiole 2, on ajoute de l'azote exogène, sous la forme d'ammoniaque à hauteur de 1 gramme d'azote par kilogramme de blé.

Pendant toute le durée des essais les moûts sont maintenus à une température de 30 °C et sous agitation à 100 rpm.

La concentration souhaitée en éthanol est de 87 g d'éthanol par litre de moût (11% v/v), ce qui correspond à un rendement de conversion amidon en éthanol de 81 %.

Le tableau 2 montre l'évolution de la concentration en éthanol dans le moût et le pH du moût en fonction de la durée de la saccharification-fermentation.

De la même façon que le tableau 1, le tableau 2 illustre que l'utilisation d'un complément nutritionnel selon l'invention dans le milieu de saccharification-fermentation permet d'obtenir une plus grande concentration en éthanol en une durée inférieure que les enzymes conventionnelles malgré un apport d'azote exogène.

De plus, le tableau 2 illustre que l'incorporation d'ammoniac dans la fiole 2 entraîne une baisse du pH autour de 2,8.

Pour obtenir un meilleur rendement en éthanol, il est important que le pH du moût reste entre 3,5 et 5. En effet, la saccharification-fermentation étant réalisée à environ 30°C, un pH trop élevé, notamment supérieur à 5, entraîne un risque de contamination du moût. Une valeur du pH trop basse, notamment inférieure à 3,5, entraîne une baisse de rendement de la fermentation alcoolique.

L'utilisation d'une préparation enzymatique à laquelle est ajoutée une source d'azote exogène dans un milieu de saccharification-fermentation nécessite un dispositif de régulation du pH, de manière à maintenir une valeur de pH entre 3,5 et 4,5.

Tableau 2

| Durée SSF (h) | Concentration en éthanol (g/l) | | pH | |
|---|---|---|---|---|
| | Son fermenté | Spirizyme + NH$_3$ | Son fermenté | Spirizyme + NH3 |
| 0 | 0 | 0 | 4 | 4 |
| 19 | 56,6 | 33,8 | 3,7 | 2,9 |
| 28 | 72,7 | 48 | | |
| 43 | 82 | 63,9 | 3,8 | 2,7 |
| 47 | 82,6 | 71 | | |
| 67 | 88 | 77,9 | 3,8 | 2,8 |

Le complément nutritionnel selon l'invention a un effet tampon sur le pH du moût de saccharification-fermentation et donc, avantageusement permet de s'affranchir d'un dispositif de régulation du pH.

Exemple 3 : Bio-stimulation

Cet exemple met en évidence l'effet de bio-stimulation du complément nutritionnel selon l'invention. Les essais de cet exemple illustrent que la complémentation en azote et l'effet de stabilisation du pH du moût autour de 4, n'expliquent que partiellement l'amélioration des performances de la levure pour la production d'éthanol lors de son introduction dans le milieu de saccharification-fermentation.

Dans ces essais, les inventeurs ont préparé du moût de farine de blé à 32 % de matière sèche. Après 1 h30 de liquéfaction à 85°C, à pH 5,5 et sous agitation à 250 rpm, le moût est réparti dans 2 bioréacteurs de 4 litres. Chaque bioréacteur contient 1,7 kg de moût à 29,8 % de matière sèche. Le moût obtenu dans chaque bioréacteur est obtenu en mélangeant le moût à 32 % de matière sèche issu de la liquéfaction avec des vinasses à 27 % de matière sèche.

Les vinasses utilisées pour diluer le moût, sont des vinasses obtenues à l'issu d'un procédé de fermentation alcoolique de blé.

Les 1,7 kg de moût à 29,8 % de matière sèche sont complétés comme suit :

➢ Bioréacteur 1 : 199 µl de Spirizyme® Fuel (solution de glucoamylase purifiée, Novozymes) et 59 µl de Viscozym Wheat® (Novozyme) (enzyme déviscosifiante) ;

➢ Bioréacteur 2: 5,7 g de son de blé fermenté par une souche *d'Aspergillus niger* ATCC 76061, suivant le procédé décrit dans US 2002 037 342.

Le pH initial des moûts dans les deux bioréacteurs a été ajusté à 4,1 et la température à 30 °C, puis les moûts sont soumis à une agitation à 100 rpm.

Les moûts sont ensuite ensemencés avec $5 \times 10^6$ UFC de levures (Ethanol Red®, Lesaffre), par ml de moût.

De l'azote exogène est ajouté dans le bioréacteur 1, de manière fractionnée, sous la forme d'ammoniaque pour arriver à une teneur de 1 gramme d'azote par kilogramme de blé.

Les vinasses issues de moût de céréales fermentées contiennent une certaine quantité de fibres. En les mélangeant au moût, on obtient un effet tampon du pH du moût.

La concentration souhaitée en éthanol est de 87 g d'éthanol par litre de moût, ce qui correspond à un rendement de conversion de l'amidon en éthanol de 81 %.

Le tableau 3 montre l'évolution de la concentration en éthanol dans le moût et le pH du moût en fonction de la durée de la saccharification-fermentation.

Le fractionnement de l'apport en azote exogène ainsi que l'utilisation des vinasses permet une stabilisation du pH dans le bioréacteur 1 autour de 4.

Comme le montre le tableau 3, la concentration souhaitée en éthanol est presque atteinte en 28 heures dans le bioréacteur avec un complément nutritionnel selon l'invention alors qu'il faut attendre 44 heures pour atteindre une concentration équivalente dans le bioréacteur avec la préparation enzymatique. De plus, au bout de 68 heures la concentration en éthanol est toujours plus élevée dans le bioréacteur avec le complément nutritionnel selon l'invention que dans celui avec la préparation enzymatique.

Le tableau 3 illustre l'avantage significatif de l'utilisation d'un complément nutritionnel selon l'invention par rapport aux préparations enzymatiques classiques. Les résultats de ces essais indiquent qu'une complémentation en azote et un contrôle du pH n'expliquent que partiellement les performances du complément nutritionnel selon l'invention.

Ces essais mettent en évidence un effet de bio-stimulation des levures lié à la composition du complément nutritionnel selon l'invention.

Tableau 3

| Durée SSF (h) | Concentration en éthanol (g/l) | | pH | |
|---|---|---|---|---|
| | Son fermenté | Spirizyme + NH$_3$ + VW | Son fermenté | Spirizyme + NH$_3$ + VW |
| 0 | 0 | 0 | 4,1 | 4,1 |
| 8 | 6,8 | < 4 | 4 | 4.1 |
| 20 | 69 | 35,5 | 3,9 | 3,75 |
| 24 | 73,5 | 48,8 | 4 | 3,8 |
| 28 | 82,5 | 58,5 | 4 | 3,9 |
| 44 | 85,6 | 82,6 | 4,1 | 3,8 |
| 48 | 93,6 | 88,9 | 4,1 | 3,85 |
| 68 | 97,1 | 91,3 | 4,2 | 4 |

Exemple 4 : Fermentation aérobie des vinasses

Les inventeurs ont étudié de façon comparative l'évolution de la concentration du milieu de pré-fermentation en levure en fonction de la teneur en matière sèche dudit milieu.

Ces essais ont été effectués sur 100 g de milieu de pré-fermentation dans des fioles à baffles de 250 ml. Les milieux de pré-fermentation sont thermostatés à 30°C, placés sous agitation à 130 rpm et ensemencés avec $2,5 \times 10^7$ levures (Ethanol Red®), par ml de milieu. Le pH initial des milieux est de 4,2 à 4,4

Chaque milieu de pré-fermentation se compose comme suit, en pourcentage massique :

➢ Fiole 1 : 1,2 % de vinasse à 28 % de matière de sèche, et environ 98,8 % d'eau ;

➢ Fiole 2 : 10 % de vinasse à 28 % de matière de sèche, et environ 90 % d'eau ;

➢ Fiole 3 : 20 % de vinasse à 28 % de matière de sèche, et environ 80 % d'eau ;

➢ Fiole 4 : 40 % de vinasse à 28 % de matière de sèche, et environ 60 % d'eau ;

➢ Fiole 5 : milieu témoin (glucose et extrait de levure).

Les fioles sont agitées de manière à oxygéner le milieu et permettre la propagation de la levure.

Les inventeurs ont mesuré l'évolution de la concentration en levures dans chaque milieu de pré-fermentation.

Les compositions des milieux dans les fioles et les évolutions des concentrations en levure sont récapitulées dans le tableau 4 suivant :

Tableau 4

| Durée de culture (h) | Dénombrement (levures/ml) | | | | |
|---|---|---|---|---|---|
| | 1,2 % vinasses | 10% vinasses | 20% vinasses | 40% vinasses | Témoin |
| 0 | 2,50E+07 | 2,50E+07 | 2,50E+07 | 2,50E+07 | 2,50E+07 |
| 5 | 3,30E+07 | 4,90E+07 | 1,54E+08 | 4,15E+08 | 4,90E+07 |
| 7 | 3,00E+07 | 2,50E+08 | 3,10E+08 | 4,50E+08 | 6.00E+07 |
| 24 | 3,30E+07 | 1,90E+08 | 3,50E+08 | 5,50E+08 | 2.00E+08 |

Comme le montre le tableau 4, un milieu de pré-fermentation composé à 40 % en masse de vinasse à 28 % en masse de matière sèche, soit un milieu à 11,2 % en masse de matière sèche, permet une multiplication d'un facteur 16,6 de la quantité de levure en 5 heures. A titre de comparaison, le facteur multiplicatif sur la même période dans le milieu témoin est d'environ 2.

Après 24 heures de culture aérobie, la concentration en levure dans le milieu composé de 40 % en masse de vinasse est plus de 2,5 fois la concentration de levure dans le milieu témoin.

Sans être liés par une théorie, les inventeurs expliquent ces résultats par la présence dans la matière sèche des vinasses de sources de carbone, autre que le glucose, assimilables par la levure. Les inventeurs ont montré que la matière sèche des vinasses contient environ 10 % de sucres fermentescibles, essentiellement sous forme de glucose ou d'amidon résiduel. La concentration initiale en levure correspond à environ 0,62 g de levure par litre de milieu, alors que la concentration finale correspond à environ 13,8 g de levure par litre de milieu. Il s'est donc formé environ 13,2 g de levure par litre de milieu, ce qui correspond à une consommation d'environ 26,4 g de glucose. Or dans la fiole n° 4 la concentration initiale en glucose est d'environ 11,2 g par litre de milieu. La quantité de levure obtenue est donc liée à une autre source de carbone que le glucose, par exemple la présence de glycérol dans les vinasses.

Bien entendu, la présente invention n'est pas limitée aux modes de réalisation décrits et représentés fournis à titre d'exemples illustratifs et non limitatifs.

## Revendications

1. Utilisation d'un complément nutritionnel dans la fabrication d'éthanol à partir d'une matière première amylacée par saccharification-fermentation,

   le complément nutritionnel étant un complément nutritionnel pour milieu de fermentation alcoolique à partir de matières premières glucidiques, comportant un principe actif, sous forme brute ou extraite, issu de la fermentation d'un son de blé avec une moisissure choisie parmi les souches d'*Aspergillus niger* ATCC 201202, ATCC 76060, ATCC 76061, MUCL 28815, MUCL 28816, NRRL 3112 ou parmi les souches d'*Aspergillus orizae* ATCC 22788 et ATCC 42149.

2. Utilisation d'un complément nutritionnel selon la revendication 1, le complément nutritionnel présentant au moins une des activités enzymatiques suivantes :

   ■ glucoamylasique : au moins 500 UG, par gramme de matière sèche,
   ■ protéolytique : au moins 100 UP par gramme de matière sèche,
   ■ xylanasique : au moins 100 UX par gramme de matière sèche.

3. Utilisation d'un complément nutritionnel selon la revendication 1 ou 2, le complément nutritionnel étant susceptible d'être obtenu au moyen d'un procédé comprenant les étapes suivantes :

   i) prendre du son de blé,
   ii) humidifier, acidifier à un pH compris entre 4 et 5 et traiter thermiquement ledit son de façon à le pasteuriser ou le stériliser,
   iii) inoculer le son de blé résultant par une souche *d'Aspergillus niger,* choisie parmi ATCC 201202, ATCC 76060, ATCC 76061, MUCL 28815, MUCL 28816, NRRL 3112, ou par une souche *d'Aspergillus orizae* choisie parmi ATCC 22788 et ATCC 42149,
   iv) faire fermenter le son de blé à l'état solide dans un réacteur agité périodiquement, à une température de 28°C à 38°C, la teneur en humidité étant maintenue enter 45% et 65% en poids, dans des conditions d'aération propres à éviter une accumulation de dioxyde de carbone permanente au sein du son de blé, jusqu'à ce que le produit de fermentation présente les valeurs minimales suivantes d'activités enzymatiques

   - glucoamylasique : au moins 500 UG UG par gramme de matière sèche et, éventuellement,
   - protéolytique : au moins 100 UP par gramme de matière sèche,
   - xylanasique : au moins 100 UX par gramme de matière sèche.

4. Utilisation d'un complément nutritionnel selon l'une quelconque des revendications 1 à 3, dans laquelle l'activité glucoamylasique du complément nutritionnel est d'au moins 750 UG par gramme de matière sèche.

5. Utilisation d'un complément nutritionnel selon la revendication 4, dans laquelle l'activité glucoamylasique du complément nutritionnel est d'au moins 1500 UG par gramme de matière sèche.

6. Utilisation d'un complément nutritionnel selon l'une quelconque des revendications 1 à 5, ledit complément nutritionnel comprenant de l'ergostérol, de la N-acétylglucosamine, des vitamines, des acides nucléiques et des acides aminés.

7. Utilisation d'un complément nutritionnel selon l'une quelconque des revendications 1 à 6, ledit complément nutritionnel comprenant de la vitamine B.

8. Utilisation d'un complément nutritionnel selon l'une quelconque des revendications 1 à 7, ledit complément nutritionnel comprenant un ou des acides aminés choisis parmi : alanine, arginine, asparagine, acide aspartique, valine, leucine, acide glutamique.

9. Procédé de fabrication d'éthanol à partir d'une matière glucidique qui est une matière amylacée, comprenant une étape de fermentation en présence d'un complément nutritionnel, et comportant, après une étape de liquéfaction et éventuellement une étape de pré-saccharifcation, une étape de saccharification-fermentation dans un milieu de saccharification-fermentation dont la teneur en glucose au début de l'étape de saccharification fermentation est au plus égal à 95% de l'équivalent en glucose correspondant à l'amidon de ladite matière amylacée, dans lequel ledit complément nutritionnel est un complément nutritionnel, pour milieu de fermentation alcoolique à partir de matières premières glucidiques, comportant un principe actif, sous forme brute ou extraite, issu de la fermentation d'un son de blé avec une moisissure choisie parmi les souches *d'Aspergillus niger* ATCC 201202, ATCC 76060, ATCC 76061, MUCL 28815, MUCL 28816, NRRL 3112 ou parmi les souches *d'Aspergillus orizae* ATCC 22788 et ATCC 42149, **caractérisé en ce qu'**on introduit le complément nutritionnel dans le milieu de saccharification-fermentation.

10. Procédé selon la revendication 9, dans lequel le procédé ne comprend pas d'étape de pré-saccharification et la teneur en glucose au début de l'étape de saccharification fermentation est au plus égale à 3% de l'équivalent en glucose correspondant à l'amidon de ladite matière amylacée.

11. Procédé selon la revendication 9, dans lequel la teneur en glucose au début de l'étape de saccharification-fermentation est au plus égal à 3% de l'équivalent en glucose correspondant à l'amidon de ladite matière amylacée.

12. Procédé selon l'une quelconque des revendications 9 à 11, le complément nutritionnel étant introduit sous forme brute, dans lequel la quantité dudit complément nutritionnel est supérieure ou égale à 4 kg de matière sèche et/ou inférieure ou égale à 60 kg de matière sèche par tonne d'amidon.

13. Procédé selon l'une quelconque des revendications 9 à 12, dans lequel ledit complément nutritionnel constitue la principale source nutritionnelle pour la levure du milieu de saccharification-fermentation et/ou la principale source d'ergostérol et/ou la principale source d'azote et/ou acides aminés et/ou de soufre et/ou de vitamines.

14. Procédé selon l'une quelconque des revendications 9 à 13, dans lequel la saccharification-fermentation est entièrement réalisée en l'absence d'apport d'air ou d'oxygène.

15. Procédé selon l'une quelconque des revendications 9 à 13, dans lequel la saccharification-fermentation comporte une phase initiale aérobie.

16. Procédé selon l'une quelconque des revendications 9 à 13, dans lequel la saccharification-fermentation est conduite dans les conditions suivantes :

- température : de 30°C à 35°C,
- pH : ajusté au début de l'étape de saccharification-fermentation entre environ 3,5 et environ 5,
- inoculum de levure : environ $10^8$ à environ $5.10^8$ UFC de levure par ml de milieu de saccharification-fermentation,
- 20% à 35% de MS,
- temps de séjour : 20 heures à 72 heures.

17. Drêches issues d'un procédé de fabrication d'éthanol selon l'une quelconque des revendications 9 à 16 comportant plus de 5 g d'ergostérol par tonne de matières sèches de drêches.

18. Drêches selon la revendication 17 comportant plus de 40%, de préférence plus de 50% de protéines brutes, en pourcentages sur la base de la matière sèche.

19. Drêches selon la revendication 17 comportant plus de 50% de protéines brutes, en pourcentages sur la base de la matière sèche.

20. Procédé de fabrication de levure en aérobiose dans un pré-fermenteur, **caractérisé en ce qu'**on ajoute dans ledit pré-fermenteur des vinasses obtenues lors de la mise en oeuvre d'un procédé de fabrication d'éthanol selon l'une quelconque des revendications 9 à 16.

21. Procédé de fabrication de levure selon la revendication 20, dans lequel les vinasses ajoutées sont des vinasses clarifiées et/ou des vinasses concentrées.

22. Procédé de fabrication d'éthanol selon l'une quelconque des revendications 9 à 16, dans lequel on utilise, pour la fermentation éthanolique, des levures obtenues par un procédé de fabrication de levure selon la revendication 20 ou 21, lequel procédé utilise des vinasses provenant dudit procédé de fabrication d'éthanol.

23. Procédé de fabrication d'éthanol selon la revendication 22, dans lequel lesdites vinasses sont des vinasses clarifiées et/ou des vinasses concentrées.


**Patentansprüche**

1. Verwendung eines Nahrungsergänzungsmittels bei der Herstellung von Ethanol aus einem stärkehaltigen Ausgangsmaterial durch Verzuckerung-Fermentation, wobei das Nahrungsergänzungsmittel ein Nahrungsergänzungsmittel für ein Milieu der alkoholischen Fermentation aus kohlenhydrathaltigen Ausgangsmaterialien ist, das einen Wirkstoff, in roher oder extrahierter Form, umfasst, der aus der Fermentation einer Weizenkleie mit einem Schimmelpüz, ausgewählt unter den Aspergillus niger-Stämmen ATCC 201202, ATCC 76060, ATCC 76061, MUCL 28815, MUCL 28816, NRRL 3112 oder unter den Aspergillus orizae-Stämmen ATCC 22788 und ATCC 42149, stammt.

2. Verwendung eines Nahrungsergänzungsmittels gemäß Anspruch 1, wobei das Nahrungsergänzungsmittel wenigstens eine der folgenden enzymatischen Aktivitäten aufweist:

   • Glucoamylase: mindestens 500 UG pro Gramm Trockensubstanz,
   • Protease: mindestens 100 UP pro Gramm Trockensubstanz,
   • Xylanase: mindestens 100 UX pro Gramm Trockensubstanz.

3. Verwendung eines Nahrungsergänzungsmittels gemäß Anspruch 1 oder 2, wobei das Nahrungsergänzungsmittel geeignet ist, mit Hilfe eines Verfahrens erhalten zu werden, das die folgenden Stufen umfasst:

   i) Bereitstellen von Weizenkleie,
   ii) Anfeuchten, Ansäuern auf einen pH zwischen 4 und 5 und Wärmebehandeln der Kleie, um sie zu pasteurisieren oder zu sterilisieren,
   iii) Inokulieren der resultierenden Weizenkleie mit einem Aspergillus niger-Stamm, ausgewählt aus ATCC 201202, ATCC 76060, ATCC 76061, MUCL 28815, MUCL 28818, NRRL 3122 oder mit einem Aspergillus orizae-Stamm, ausgewählt ATCC 22788 und ATCC 42149,
   iv) Fermentierenlassen der Weizenkleie in festem Zustand in einem Reaktor, der in regelmäßigen Abständen bewegt wird, bei einer Temperatur von 28 °C bis 38 °C, wobei der Feuchtigkeitsgehalt zwischen 45 und 65 Gew.-% gehalten wird, unter geeigneten Belüftungsbedingungen, um eine permanente Ansammlung von Kohlendioxid im Inneren der Weizenkleie zu vermeiden, bis das Fermentationsprodukt die folgenden Mindestwerte von Enzymaktivitäten aufweist:

   - Glucoamylase: mindestens 500 UG pro Gramm Trockensubstanz und, gegebenenfalls,
   - Protease: mindestens 100 UP pro Gramm Trockensubstanz,
   - Xylanase: mindestens 100 UX pro Gramm Trockensubstanz.

4. Verwendung eines Nahrungsergänzungsmittels gemäß einem der Ansprüche 1 bis 3, wobei die Glucoamylase-Aktivität des Nahrungsergänzungsmittels mindestens 750 UG pro Gramm Trockensubstanz ist.

5. Verwendung eines Nahrungsergänzungsmittels gemäß Anspruch 4, wobei die Glucoamylase-Aktivität des Nahrungsergänzungsmittels mindestens 1500 UG pro Gramm Trockensubstanz ist.

6. Verwendung eines Nahrungsergänzungsmittels gemäß einem der Ansprüche 1 bis 5, wobei das Nahrungsergänzungsmittel Ergosterol, N-Acetylglucosamin, Vitamine, Nucleinsäuren und Aminosäuren umfasst.

7. Verwendung eines Nahrungsergänzungsmittels gemäß einem der Ansprüche 1 bis 6, wobei das Nahrungsergänzungsmittels Vitamin B umfasst.

8. Verwendung eines Nahrungsergänzungsmittels gemäß einem der Ansprüche 1 bis 7, wobei das Nahrungsergänzungsmittel (eine) Aminosäure(n), ausgewählt aus Alanin, Arginin, Asparagin, Asparaginsäure, Valin, Leucin, Glutaminsäure, umfasst.

9. Verfahren zur Herstellung von Ethanol aus einem kohlenhydrathaltigen Material, welches ein stärkehaltiges Material ist, das eine Fermentationsstufe in Gegenwart eines Nahrungsergänzungsmittels umfasst und das nach einer Stufe der Verflüssigung und gegebenenfalls einer Stufe der Vorverzuckerung eine Stufe der Verzuckerung-Fermentation in einem Milieu der Verzuckerung-Fermentation umfasst, dessen Glucosegehalt zu Beginn der Stufe der Verzuckerung-Fermentation höchstens 95 % des Äquivalents an Glucose, das der Stärke des stärkehaltigen Materials entspricht, ist, wobei das Nahrungsergänzungsmittel ein Nahrungsergänzungsmittel für ein Milieu der alkoholischen Gärung aus kohlenhydrathaltigen Ausgangsmaterialien ist, das einen Wirkstoff, in roher oder Extraktform, enthält, der aus der Gärung einer Weizenkleie mit einem Schimmelpilz, ausgewählt aus den Aspergillus niger-Stämmen ATCC 201202, ATCC 76060, ATCC 76061, MUCL 28815, MUCL 28816, NRRL 3112 oder den Aspergillus orizae-Stämmen ATCC 22788 und ATCC 42149, stammt,
**dadurch gekennzeichnet, dass** man das Nahrungsergänzungsmittel in das Milieu der Verzuckerung-Fermentation einführt.

10. Verfahren gemäß Anspruch 9, wobei das Verfahren keine Stufe der Vorverzuckerung umfasst und der Gehalt an Glucose zu Beginn der Stufe der Verzuckerung-Fermentation höchstens 3 % des Äquivalents an Glucose, das der Stärke des stärkehaltigen Materials entspricht, ist.

11. Verfahren gemäß Anspruch 9, in dem der Gehalt an Glucose zu Beginn der Stufe der Verzuckerung-Fermentation höchstens 3 % des Äquivalents an Glucose, das der Stärke des stärkehaltigen Materials entspricht, ist.

12. Verfahren gemäß einem der Ansprüche 9 bis 11, wobei das Nahrungsergänzungsmittel in roher Form eingeführt wird, in dem die Menge des Nahrungsergänzungsmittels über oder gleich 4 kg Trockensubstanz und/oder unter oder gleich 60 kg Trockensubstanz pro Tonne Stärke ist.

13. Verfahren gemäß einem der Ansprüche 9 bis 12, in dem das Nahrungsergänzungsmittel die Hauptnahrungsquelle für die Hefe des Milieus der Verzuckerung-Fermentation und/oder die Hauptquelle für Ergosterol und/oder die Hauptquelle für Stickstoff und/oder Aminosäuren und/oder Schwefel und/oder Vitamine bildet.

14. Verfahren gemäß einem der Ansprüche 9 bis 13, in dem die Verzuckerung-Fermentation vollständig in Abwesenheit einer Zufuhr von Luft oder Sauerstoff durchgeführt wird.

15. Verfahren gemäß einem der Ansprüche 9 bis 13, in dem die Verzuckerung-Fermentation eine anaerobe Anfangsphase umfasst.

16. Verfahren gemäß einem der Ansprüche 9 bis 13, in dem die Verzuckerung-Fermentation unter den folgenden Bedingungen durchgeführt wird:

- Temperatur: 30 °C bis 35 °C,
- pH: eingestellt am Anfang der Stufe der Verzuckerung-Fermentation auf zwischen etwa 3,5 und etwa 5,
- Hefeinokulum: etwa $1^6$ bis etwa $5 \times 10^8$ KbE Hefe pro ml des Milieus der Verzuckerung-Fermentation,
- 20 % bis 35 % MS,
- Verweildauer: 20 Stunden bis 72 Stunden.

17. Treber, die aus einem Verfahren zur Herstellung von Ethanol gemäß einem der Ansprüche 9 bis 16 stammen, die mehr als 5 g Ergosterol pro Tonne Treber-Trockensubstanz umfassen.

18. Treber gemäß Anspruch 17, die mehr als 40 %, vorzugsweise mehr als 50 % Rohproteine, als Prozentwerte auf der Basis der Trockensubstanz, umfassen.

19. Treber gemäß Anspruch 17, die mehr als 50 % Rohproteine, als Prozentwerte auf der Basis der Trockensubstanz, umfassen.

20. Anaerobes Verfahren zur Herstellung von Hefe in einem Vorfermenter, **dadurch gekennzeichnet, dass** man in den genannten Vorfermenter Schlempen gibt, die während Durchführung eines Verfahrens zur Herstellung von Ethanol gemäß einem der Ansprüche 9 bis 16 erhalten wurden.

21. Verfahren zur Herstellung von Hefe gemäß Anspruch 20, in dem die zugesetzten Schlempen geklärte Schlempen und/oder konzentrierte Schlempen sind.

22. Verfahren zur Herstellung von Ethanol gemäß einem der Ansprüche 9 bis 16, in dem man für die ethanolische Fermentation die Hefen verwendet, die durch ein Verfahren zur Herstellung von Hefe gemäß Anspruch 20 oder 21 erhalten wurden, wobei das Verfahren Schlempen verwendet, die aus dem Verfahren zur Herstellung von Ethanol stammen.

23. Verfahren zur Herstellung von Ethanol gemäß Anspruch 22, in dem die genannten Schlempen geklärte Schlempen und/oder konzentrierte Schlempen sind.

**Claims**

1. Use of a nutritional supplement for the manufacture of ethanol from starchy raw material by saccharification-fermentation, the nutritional supplement being a nutritional supplement for alcoholic fermentation medium from carbohydrate raw materials, comprising an active ingredient, in crude or extracted form, derived from fermentation of a wheat bran with a mold chosen from the *Aspergillus niger* strains ATCC 201202, ATCC 76060, ATCC 76061, MUCL 28815, MUCL 28816, NRRL 3112 or from the *Aspergillus orizae* strains ATCC 22788 and ATCC 42149.

2. Use of a nutritional supplement according to claim 1, the nutritional supplement having at least one of the following enzymatic activities:

   ■ glucoamylase: at least 500 GU, per gram of dry matter,
   ■ proteolytic: at least 100 PU per gram of dry matter,
   ■ xylanase: at least 100 XU per gram of dry matter.

3. Use of a nutritional supplement according to claim 1 or 2, the nutritional supplement being susceptible to be obtained by means of a process comprising the following steps:

   i) taking wheat bran,
   ii) moistening, acidifying to a pH of between 4 and 5 and heat treating said bran so as to pasteurize it or sterilize it,
   iii) inoculating the resulting wheat bran with an *Aspergillus niger* strain, chosen from ATCC 201202, ATCC 76060, ATCC 76061, MUCL 28815, MUCL 28816, NRRL 3112, or with an *Aspergillus orizae* strain chosen from ATCC 22788 and ATCC 42149,
   iv) fermenting the wheat bran in the solid state in a reactor which is periodically stirred, at a temperature of 28°C to 38°C, the moisture content being maintained between 45% and 65% by weight, under aeration conditions designed to avoid a permanent accumulation of carbon dioxide in the wheat bran, until the fermentation product has the following minimum enzyme activity values:

   ■ glucoamylase: at least 500 GU per gram of dry matter and, optionally,
   ■ proteolytic: at least 100 PU per gram of dry matter,
   ■ xylanase: at least 100 XU per gram of dry matter.

4. Use of a nutritional supplement according to any one of claims 1 to 3, wherein the glucoamylase activity of the nutritional supplement is at least 750 GU per gram of dry matter.

5. Use of a nutritional supplement according to claim 4, wherein the glucoamylase activity of the nutritional supplement is at least 1500 GU per gram of dry matter.

6. Use of a nutritional supplement according to any one of claims 1 to 5, the nutritional supplement comprising ergosterol, N-acetylglucosamine, vitamins, nucleic acids, and amino acids.

7. Use of a nutritional supplement according to any one of claims 1 to 6, the nutritional supplement comprising vitamin B.

8. Use of a nutritional supplement according to any one of claims 1 to 7, the nutritional supplement comprising one or more amino acids chosen from: alanine, arginine, asparagine, aspartic acid, valine, leucine and glutamic acid.

9. Process for the manufacture of ethanol from carbohydrate material which is a starchy material, comprising a step of fermentation in presence of a nutritional supplement, and comprising, after a liquefaction step and optionally a presaccharification step, a simultaneous saccharification and fermentation step in a simultaneous saccharification and fermentation medium whose glucose content at the start of the simultaneous saccharification and fermentation step is at most equal to 95% of the glucose equivalent corresponding to the starch of said starchy material, wherein the nutritional supplement is a nutritional supplement for alcoholic fermentation medium from carbohydrate raw materials, comprising an active ingredient, in crude or extracted form, derived from fermentation of a wheat bran with a mold chosen from the *Aspergillus niger* strains ATCC 201202, ATCC 76060, ATCC 76061, MUCL 28815, MUCL 28816, NRRL 3112 or from the *Aspergillus orizae* strains ATCC 22788 and ATCC 42149, **characterized in that** the nutritional supplement is introduced into the simultaneous saccharification and fermentation medium.

10. Process according to claim 9, wherein the process does not comprise a presaccharification step and the glucose content at the start of the simultaneous saccharification and fermentation step is at most equal to 3% of the glucose equivalent corresponding to the starch of said starchy material.

11. Process according to claim 9, wherein the glucose content at the start of the simultaneous saccharification and fermentation step is at most equal to 3% of the glucose equivalent corresponding to the starch of said starchy material.

12. Process according to any one of claims 9 to 11, the nutritional supplement being introduced in crude form, wherein the quantity of said nutritional supplement is greater than or equal to 4 kg of dry matter and/or less than or equal to 60 kg of dry matter per ton of starch.

13. Process according to any one of claims 9 to 12, wherein said nutritional supplement constitutes the main nutritional source for the yeast of the simultaneous saccharification and fermentation medium and/or the main source of ergosterol and/or the main source of nitrogen and/or of amino acids and/or of sulfur and/or of vitamins.

14. Process according to any one of claims 9 to 13, wherein the simultaneous saccharification and fermentation is entirely carried out in the absence of an air or oxygen supply.

15. Process according to any one of claims 9 to 13, wherein the simultaneous saccharification and fermentation comprises an initial aerobic phase.

16. Process according to any one of claims 9 to 13, wherein the simultaneous saccharification and fermentation is performed under the following conditions:

- temperature: from 30°C to 35°C,
- pH: adjusted at the start of the simultaneous saccharification and fermentation step to between about 3.5 and about 5,
- yeast inoculum: about $10^6$ to about $5 \times 10^8$ CFU of yeast per ml of simultaneous saccharification and fermentation medium,
- 20% to 35% of DM,
- residence time: 20 hours to 72 hours.

17. Brewers' grain derived from a process for the manufacture of ethanol according to any one of claims 9 to 16, containing more than 5 g of ergosterol per ton of dry matter of brewers' grain.

18. Brewers' grain according to claim 17, containing more than 40%, preferably more than 50% of crude proteins, as percentages on the basis of the dry matter.

19. Brewers' grain according to claim 17, containing more than 50% of crude proteins, as percentages on the basis of the dry matter.

20. Process for the manufacture of yeast under aerobic conditions in a prefermenter, **characterized in that** there are added to said prefermenter vinasses obtained during the carrying out of a process for the manufacture of ethanol according to any one of claims 9 to 16.

21. Process for the manufacture of yeast according to claim 20, wherein the added vinasses are clarified vinasses and/or concentrated vinasses.

22. Process for the manufacture of ethanol according to any one of claims 9 to 16, wherein yeasts obtained by a process for the manufacture of yeast according to claim 20 or 21 are used for the ethanol fermentation, which process uses vinasses obtained from said process for the manufacture of ethanol.

23. Process for the manufacture of ethanol according to claim 22, wherein said vinasses are clarified vinasses and/or concentrated vinasses.

Fig. 1

a) Conditionnement

b) Liquéfaction

c) Pré-saccharification

Pré-fermentation

Levure

d) Saccharification-Fermentation

Distillation ----> Azéotrope éthanol – eau

Vinasses clarifiées <---- Séparation

Concentration

Vinasses concentrées <---- Séchage

Drêches

Fig. 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1022329 A

- US 2002037342 A

**Littérature non-brevet citée dans la description**

- **TANAKA.** *Appl. Microbiol. Biotechnnol.,* 2006, vol. 69, 627-642
- **A. DURAND.** *Agro-Food-Industry Hi-Tech,* Mai 1997, 39-42
- **P. BEMFELD.** *Methods in enzymology,* 1955, vol. 1, 149-158
- Proteins Purification Methods - a Practical Approach. IRL-Press, Oxford University Press, 1989, 1-66